# EUROPEAN PATENT SPECIFICATION

(11) **EP 1 663 233 B1**
(45) Date of publication and mention of the grant of the patent: **23.07.2008**
(21) Application number: 04783278.7
(22) Date of filing: 07.09.2004
(51) Int. Cl.: A61K 31/496, A61P 19/02, A61P 31/04, A61K 31/65

(54) **USE OF TIGECYCLINE, ALONE, OR IN COMBINATION WITH RIFAMPIN TO TREAT OSTEOMYELITIS AND/OR SEPTIC ARTHRITIS**
VERWENDUNG VON TIGECYCLIN, ALLEIN ODER IN KOMBINATION MIT RIFAMPIN ZUR BEHANDLUNG VON OSTEOMYELITIS UND/ODER SEPTISCHER ARTHRITIS
TRAITEMENT DE L'OSTEOMYELITE ET/OU DE L'ARTHRITE PURULENTE A BASE DE TIGECYCLINE, SEULE, OU ASSOCIE A LA RIFAMPINE

(30) Priority: 05.09.2003 US 500474 P; 03.09.2004 US 933455 P
(43) Date of publication of application: 07.06.2006
(73) Proprietor: Wyeth, Madison, New Jersey 07940 (US); BOARD OF REGENTS, THE UNIVERSITY OF TEXAS SYSTEM, Austin, TX 78701 (US)
(72) Inventor: TESTA, Raymond, Thomas, Cedar Grove, New Jersey 07009 (US); CALHOUN, Jason Department of Orthopaedic Surgery, Suite MC213 Columbia, Missouri 65212 (US); MADER, Jon, T., deceased (US)
(74) Representative: Neuefeind, Regina
(86) International application number: PCT/US2004/028980
(87) International publication number: WO 2005/023263

(56) References cited:
- WO-A-02/102384
- WO-A-03/005971
- WO-A-03/099217
- NORDEN C W ET AL: "Chronic staphylococcal osteomyelitis: treatment with regimens containing rifampin." REVIEWS OF INFECTIOUS DISEASES. 1983 JUL-AUG, vol. 5 Suppl 3, July 1983 (1983-07), pages S495-S501, XP009041854 ISSN: 0162-0886
- MERCIER RENEE-CLAUDE ET AL: "Antimicrobial activity of tigecycline (GAR-936) against Enterococcus faecium and Staphylococcus aureus used alone and in combination." PHARMACOTHERAPY, vol. 22, no. 12, December 2002 (2002-12), pages 1517-1523, XP009041884 ISSN: 0277-0008
- PATEL R ET AL: "In vitro activity of GAR-936 against vancomycin-resistant enterococci, methicillin-resistant Staphylococcus aureus and penicillin-resistant Streptococcus pneumoniae." DIAGNOSTIC MICROBIOLOGY AND INFECTIOUS DISEASE. NOV 2000, vol. 38, no. 3, November 2000 (2000-11), pages 177-179, XP002311637 ISSN: 0732-8893
- ZIMMERLI W ET AL: "Role of rifampin for treatment of orthopedic implant-related staphylococcal infections: a randomized controlled trial. Foreign-Body Infection (FBI) Study Group." JAMA : THE JOURNAL OF THE AMERICAN MEDICAL ASSOCIATION. 20 MAY 1998, vol. 279, no. 19, 20 May 1998 (1998-05-20), pages 1537-1541, XP009041853 ISSN: 0098-7484
- LEW D P ET AL: "Osteomyelitis." THE NEW ENGLAND JOURNAL OF MEDICINE. 3 APR 1997, vol. 336, no. 14, 3 April 1997 (1997-04-03), pages 999-1007, XP009041924 ISSN: 0028-4793
- VARIOUS: "The Merck Index, 7th Ed." 1999, MERCK RESEARCH LABORATORIES , WHITEHOUSE STATION, NJ, USA , XP002311638 page 455 - page 460

## Description

### FIELD OF THE INVENTION

The present invention relates to a novel method of treating osteomyelitis and septic arthritis caused by or as a result of bacterial infections. The present invention also relates to treatment of bacterial infections of the bone, bone marrow, joint, and synovial fluid. The present invention further relates to treatment of antibiotic resistant bacterial infections in these diseases and tissues.

### BACKGROUND OF INVENTION

The last half of the 20^{th} century saw significant progress in the development of antibacterial agents. This success fostered the perception that bacterial diseases were more readily cured than any other major disorder, but the emergence of multidrug-resistant organisms in the 1990s resulted in serious public health implications. Resistance has spread to previously susceptible organisms, and some organisms are essentially resistant to all approved antibacterial agents.

Tigecycline, which belongs to the glycylcycline class of antibiotics, circumvents existing mechanisms of microbial resistance. It demonstrates a broad spectrum of antibacterial activity, inhibiting multiple resistant gram-positive, gram-negative, and anaerobic bacteria. Tigecycline is active against most common pathogens. Tigecycline is active against pathogens such as methicillin-resistant *Staphylococcus aureus* (MRSA), vancomycin-resistant enterococci (including *Enterococcus faecalis),* penicillin-resistant/macrolide-resistant pneumococci, *Prevotella* spp., peptostreptococci, mycobacteria, and minocycline-resistant organisms (Boucher et al., Antimicrob Agents Chemother. 2000; 44(8): 2225-2229, Gales et al., Antimicrob Agents Chemother. 2000; 46: 19-36, Goldstein et al., Antimicrob Agents Chemother. 2000; 44(10): 2747-2751). Tigecycline is useful in the treatment of respiratory pathogens such as *Streptococcus pneumoniae* (penicillin sensitive and penicillin resistant), *Haemophilus influenzae, Chlamydia pneumoniae, Mycoplasma pneumoniae, Staphylococcus aureus* (methicillin-susceptible and methicillin-resistant), aerobic gram-negative rods, and enterococci (vancomycin-susceptible and vancomycin-resistant enterococci). The *in vivo* results have been very encouraging and better than would be predicted based on time above minimum inhibitory concentration (MIC) in serum. Tigecycline is observed to be a safe antibacterial agent.

Methicillin-resistant staphylococci are the most common organisms in infections of the bone and joint (Waldvogel, Infectious Diseases 1988: 1339-1344). The options for treatment of infections due to these microrganisms are limited: the sensitivity of clinical strains to quinolones, clindamycin, cotrimoxazole, and rifampin is variable, and the sensitivity is often limited to glycopeptides, which must be administered by the parenteral route. Resistance of staphylococci to glycopeptides has already been described and represents a major concern, since those drugs are considered the gold standard for the treatment of serious infections due to methicillin-resistant staphylococci (Smith, et al., N Engl J Med 1999; 340: 493-501).

Novel drugs for the treatment of methicillin-resistant staphylococcal infections, such as quinupristin-dalfopristin and linezolid have recently been introduced in clinical practice (Johnson, et al., Lancet 1999; 354: 2012-2013, Livermore, J Antimicrob Chemother 2000; 46: 347-350). However, none have been fully investigated in clinical studies on the treatment of osteomyelitis.

The treatment of acute and chronic orthopedic infections is difficult, due in part to the fact that many of the infections result from antibiotic resistant pathogens but also in part due to the location of the infection. Often the therapy requires a prolonged antibiotic therapy and surgical treatment (Lazzarini et al., Curr Infect Dis Rep 2002: 4: 439-445). Several studies have been performed using various animal model of osteomyelitis (Rissing, Infect Dis Clin North Am 1990; 4: 377-390). Despite a prolonged antibiotic treatment, viable bacteria may be still found in the bone. Eradication of more bacteria from the bone has been associated with a prolonged duration of antibiotic treatment (Norden, Rev Infect Dis 1988; 10: 103-110). After four weeks of antibiotic treatment, the majority of antibiotic regimens were unable to eradicate staphylococci from the bone.

Antibiotic treatment for osteomyelitis is traditionally administered by the intravenous route. However, oral regimens for osteomyelitis have been successfully tested in human trials (Bell, Lancet 1968; 10: 295-297, Feigin et al., Pediatr 1975; 55: 213-223, Slama et al., Am J Med 1987; 82 (Suppl 4A): 259-261). Unfortunately, the choice of oral antimicrobials is restricted when dealing with multi-drug resistant organisms and treatment of these multi-drug resistant organisms may require the use of parenteral drugs (Tice, Infect Dis Clin North Am 1998; 12: 903-919).

Norden et al. also reported in an earlier paper the treatment of chronic staphylococcal osteomyelitis with rifampin as a component of antibiotic therapy ("Chronic staphylococcal osteomyelitis: treatment with regiments containing rifampin", Reviews of infectious diseases, 1983, Vol. 5, Suppl. 3, pages S495-S501).

WO 03/00597 A (Paratek Pharmaceuticals, Inc., 2003) describes numerous tetracycline compounds for the treatment of a long list of diseases, for instance inflammatory process associated states, neurological disorders and neuroprotection as well as cancer and related disorders.

Mercier et al. analysed in their research paper "Antimicrobial activity of tigecycline (GAR-936) against Enterococus faecium and Staphylococcus aureus used alone and in combination" (Pharmacotherapy, Vol. 22(12), 2002, pages 1517-1523) the activity of tigecycline and rifampin as antibacterial agents and concluded that rifampin did not enhance the antimicrobial activity of tigecycline against the *S. aureus* strains tested.

The international patent application WO 02/102384 A, 27 (December 2002) discloses the primary role of bacteria for the evocation of osteoarthritis and suggests the use of antibiotics, in particular the use of doxycycline, as suitable treatment.

Patel et al. describe GAR-936 as an promising agent active against methicillin-resistant *S. aureus* strains ("In vitro activity of GAR-936 against vancomycin-resistant Enterococci, methicillin-resistant Staphylococcus aureus and penicillin-resistant Streptococcus pneumoniae", Diagnostic Microbiology and Infectious Disease, Vol. 38(3), 2000, pages 177-179).

Zimmerli et al. disclose the treatment of osteomyelitis caused by *S*. *aureus* infections subsequent to implantation of orthopaedic devices with a course of flucoxacillin/rifampin or vancomycin/rifampin followed by ciprofloxacin/rifampin long term therapy ("Role of rifampin for treatment of orthopaedic implant-related staphylococcal infections: a randomized controlled trial. Foreign-Body-Infection (FBI) Study Group" (JAMA, Vol. 279(19), 1998, pages 1537-1541).

Lew et al. give an account of *S*. *aureus* as the most frequent microorganism in any type of osteomyelitis (table 1) in their paper "Osteomyelitis" (The New England Journal of Medicine, 3 April 1997, Vol. 336(14), pages 999-1007) and recommend the use of antibiotics from the glycopeptide-group (vancomycin or teicoplanin) for the treatment of methicillin-resistant *S. aureus* strains (table 2). Alternatively, oral quinolones in combination with rifampin can be used (page 1005, second paragraph).

A list of bacteria that most commonly cause infectious arthritis and osteomyelitis is also provided by "The Merck Index, 7th Ed.", 1999, Merck Research Laboratories, Whitehouse Station, NJ, USA.

There thus remains a need for a method of treating osteomyelitis and/or septic arthritis caused by bacterial infections, especially those caused by antibiotic resistant bacterial strains. The present invention fulfills this long-standing need.

### SUMMARY OF THE INVENTION

The present invention relates to a method of treating bone or bone marrow infections (often referred to as osteomyelitis) and/or joint infections and infections of the surrounding tissues (often referred to as septic arthritis) in a mammal, preferably a human. The method comprises administering to the mammal a pharmacologically effective amount of tigecycline and optionally a second antimicrobial agent selected from the group consisting of rifamycin, rifampin, rifapentine, rifaximin, or streptovaricin to treat the infection. Preferably the second antimicrobial is rifampin.

The infection may be caused by a pathogen selected from the group consisting of gram negative bacteria, gram positive bacteria, anaerobic bacteria, and aerobic bacteria. Exemplary bacteria include *Staphylococcus, Acinetobacter Mycobacterium, Haemophilus, Salmonella, Streptococcus, Enterobacteriaceae, Enterococcus, Escherichia, Pseudomonas, Neisseria, Rickettsia, Pneumococci, Prevotella, Peptostreptococci, Bacteroides Legionella,* beta-haemolytic streptococci, group B streptococcus and *Spirochetes.* Preferably, the infection is comprised of *Neisseria, Mycobacterium, Staphylococcus* and *Haemophilus* and more preferably *Neisseria meningitidis, Mycobacterium tuberculosis, Staphylococcus aureus, Staphylococcus epidermidis, Streptococcus pyogenes, Streptococcus pneumoniae, Haemophilus influenzae,* or *Mycobacterium leprae.*

In preferred embodiments the infection is comprised of a pathogen exhibiting antibiotic resistance. Exemplary antibiotic resistance includes methicillin resistance, glycopeptide resistance, tetracycline resistance, oxytetracycline resistance, doxycycline resistance; chlortetracycline resistance, minocycline resistance, glycylcycline resistance, cephalosporin resistance, ciprofloxacin resistance, nitrofurantoin resistance, trimethoprim-sulfa resistance, piperacillin/tazobactam resistance, moxifloxacin resistance, vancomycin resistance, teicoplanin resistance, penicillin resistance, and macrolide resistance.

A preferred glycopeptide resistance is vancomycin resistance. In another preferred embodiment, the infection is comprised of *S*. *aureus* exhibiting a resistance selected from the group consisting of glycopeptide resistance, tetracycline resistance, minocycline resistance, methicillin resistance, vancomycin resistance and resistance to glycylcycline antibiotics other than tigecycline.

In another embodiment the infection is comprised of *Acinetobacter baumannii,* which may or may not exhibit antibiotic resistance selected from the group consisting of cephalosporin resistance, ciprofloxacin resistance, nitrofurantoin resistance, trimethoprim-sulfa resistance, and piperacillin/tazobactam resistance.

In another embodiment, the infection is comprised of *Mycobacterium abscessus* that may or may not exhibit moxifloxacin resistance. In other embodiments the infection is comprised of *Haemophilus influenzae, Enterococcus faecium, Escherichia coli, Neisseria gonorrhoeae, Rickettsia prowazekii, Rickettsia typhi,* or *Rickettsia rickettsii.*

The present invention also relates to the use of a pharmacologically effective amount of tigecycline for treating osteomyelitis and/or septic arthritis in a mammal. In another embodiment, the present invention relates to use of a pharmacologically effective amount of tigecycline and optionally a second antimicrobial agent selected from the group consisting of rifamycin, rifampin, rifapentine, rifaximin, or streptovaricin to treat osteomyelitis and/or septic arthritis. In another embodiment, the invention provides a use of a pharmacologically effective amount of tigecycline for manufacture of a medicament for treatment of osteomyelitis and/or septic arthritis in a mammal. In another embodiment, there is provided a use of a pharmacologically effective amount of tigecycline and optionally a second antimicrobial agent selected from the group consisting of rifamycin, rifampin, rifapentine, rifaximin, or streptovaricin for manufacture of a medicament for treatment of osteomyelitis and/or septic arthritis in a mammal.

### BRIEF DESCRIPTION OF THE DRAWINGS

Figure 1 shows the pharmacokinetics of tigecycline in normal new Zealand white rabbits, which establishes serum levels above the minimum inhibitory concentration over twelve hours after treatment with 14 mg/kg of tigecycline.
Figure 2 shows the investigators' grading of extent of bone infection as seen in x-ray images. The data demonstrate the effective treatment of osteomyelitis by tigecycline and tigecycline in combination with rifampin over controls.
Figure 3 shows the colony-forming units per gram of marrow and bone in each of the treatments, which demonstrates that tigecycline and tigecycline in combination with rifampin were an effective treatment for infection of the bone and infection of the marrow with respect to controls.
Figure 4A provides a graphic depiction of the weights of rabbits throughout the time course of administration of various antibacterials.
Figure 4B provides a graphic depiction of weight variances of rabbits throughout the time course of administration of various antibacterials.
Figures 5A and 5B show the peaks and troughs of tigecycline (14 mg/kg twice daily) and vancomycin (30 mg/kg twice daily) in the serum of infected rabbits after administration of the respective drugs. The data demonstrate that the antibiotic serum levels were above minimum inhibitory concentrations throughout treatment.

### DETAILED DESCRIPTION OF THE INVENTION

The present invention is directed to methods of treating bone and bone marrow infections in a mammal. Preferably the mammal is human. In a preferred embodiment, the bone or bone marrow infection causes osteomyelitis. Osteomyelitis is an acute or chronic infection of the bone and/or bone marrow, and includes the related inflammatory process of the bone and its structures due to infection with pyogenic organisms. The infection associated with osteomyelitis may be localized or it may spread through the periosteum, cortex, marrow, and cancellous tissue. Common bacterial pathogens causing osteomyelitis vary on the basis of the patient's age and the mechanism of infection. Acute osteomyelitis includes two primary categories: heamatogenous osteomyelitis and direct or contiguous inoculation osteomyelitis.

Heamatogenous osteomyelitis is an infection caused by bacterial seeding from the blood. Acute heamatogenous osteomyelitis is characterized by an acute infection of the bone caused by the seeding of the bacteria within the bone from a remote source. Heamatogenous osteomyelitis occurs primarily in children. The most common site is the rapidly growing and highly vascular metaphysis of growing bones. The apparent slowing or sludging of blood flow as the vessels make sharp angles at the distal metaphysis predisposes the vessels to thrombosis and the bone itself to localized necrosis and bacterial seeding. These changes in bone structure may be seen in x-ray images. Acute haematogenous osteomyelitis, despite its name, may have a slow clinical development and insidious onset.

Direct or contiguous inoculation osteomyelitis is caused by direct contact of the tissue and bacteria during trauma or surgery. Direct inoculation (contiguous-focus) osteomyelitis is an infection in the bone secondary to the inoculation of organisms from direct trauma, spread from a contiguous focus of infection, or sepsis after a surgical procedure. Clinical manifestations of direct inoculation osteomyelitis are more localized than those of haematogenous osteomyelitis and tend to involve multiple organisms/pathogens.

Additional categories include chronic osteomyelitis and osteomyelitis secondary to peripheral vascular disease. Chronic osteomyelitis persists or recurs, regardless of its initial cause and/or mechanism and despite aggressive intervention. Although listed as an etiology, peripheral vascular disease is actually a predisposing factor rather than a true cause of infection.

Symptoms of osteomyelitis often include high fever, fatigue, irritability and malaise. Often movement may be restricted in an infected limb or joint. Local edema, erythema, and tenderness generally accompany the infection and warmth may be present around the affected area. Sinus tract drainage may also be present at later stages of infection. Hematogenous osteomyelitis usually presents with a slow insidious progression of symptoms, while chronic osteomyelitis may included a non-healing ulcer, sinus tract drainage, chronic fatigue and malaise. Direct osteomyelitis generally presents with prominent signs and symptoms in a more localized area.

Certain disease states are known to predispose patients to osteomyelitis. These include diabetes mellitus, sickle cell disease, acquired immune deficiency syndrome (AIDS), IV drug abuse, alcoholism, chronic steroid use, immunosuppression, and chronic joint disease. In addition, the presence of a prosthetic orthopedic device is an independent risk factor as is any recent orthopedic surgery or open fracture.

Several bacterial pathogens are commonly known to cause acute and direct osteomyelitis. For example, acute haematogenous osteomyelitis in newborns (younger than 4 months) is frequently caused by *S. aureus, Enterobacter* species, and group A and B *Streptococcus* species. In children aged 4 months to 4 years, acute haematogenous osteomyelitis is commonly caused by *S. aureus,* group A *Streptococcus* species, *Haemophilus influenzae,* and *Enterobacter* species. In children and adolescents aged 4 years to adult, acute haematogenous osteomyelitis is commonly caused by *S. aureus* (80%), group A *Streptococcus* species, *Haemophilus influenzae,* and *Enterobacter* species. In adults, acute haematogenous osteomyelitis is commonly caused by *S. aureus* and occasionally *Enterobacter* or *Streptococcus* species. Primary treatment has in the past included a combination of penicillinase-resistant synthetic penicillin and a third-generation cephalosporin. Alternate therapy includes vancomycin or clindamycin and a third-generation cephalosporin. In addition to these above-mentioned antibacterials, ciprofloxacin and rifampin have been used in a combination therapy for adult patients. In instances where there is evidence of infection with gram-negative bacilli, a third-generation cephalosporin is often administered.

Direct osteomyelitis is commonly caused generally by *S*. *aureus, Enterobacter* species, and *Pseudomonas* species. Often times direct osteomyelitis is caused by a puncture wound through an athletic shoe. In these cases, direct osteomyelitis is commonly caused by *S. aureus* and *Pseudomonas* species. The primary antibiotics in this scenario include ceftazidime or cefepime. Ciprofloxacin is often used as an alternative treatment. In patients with sickle cell disease, direct osteomyelitis is commonly caused by *S. aureus* and *Salmonella* species, and the primary choice for treatment is a fluoroquinolone antibiotic (not in children). A third-generation cephalosporin (e.g., ceftriaxone) is an alternative choice.

For patients with osteomyelitis due to trauma, the infecting agents usually include *S. aureus, coliform bacilli,* and *Pseudomonas aeruginosa.* Primary antibiotics are nafcillin and ciprofloxacin. Alternatives include vancomycin and a third-generation cephalosporin with antipseudomonal activity.

Accordingly, as used herein and in the claims, the term "osteomyelitis" includes haematogenous osteomyelitis, direct or contiguous inoculation osteomyelitis, chronic osteomyelitis and osteomyelitis secondary to peripheral vascular disease. Osteomyelitis may be the result of infections caused by any of the above described pathogens, but also includes other pathogens having the ability to infect the bone, bone marrow, joint, or surrounding tissues.

The term "treating osteomyelitis" includes eradication of the pathogens/bacteria causing the underlying infection associated with osteomyelitis, inhibition of bacterial growth, reduction in bacterial concentration, reduction in recovery time from infection, improvement, elimination, or reduction of symptoms of infection such as swelling, necrosis, fever, pain, weakness, and or other indicators as are selected as appropriate measures by those skilled in the art.

Another embodiment of the present invention relates to methods of treating joint infections and/or surrounding tissue infections in a mammal. Preferably the mammal is human. In a preferred embodiment, the joint infection and/or surrounding tissue infection causes septic arthritis.

Septic arthritis is an infection of the joint and surrounding tissues and results in joint inflammation caused by the presence of live intra-articular micro-organisms. Septic arthritis most commonly occurs secondary to osteomyelitis, especially in childhood, and arises as a result of bacterial infection.

Infection of the joint can occur by several routes. Most commonly, the spread of the infecting pathogen is haematogenous. Frequently septic arthritis arises from infections or abscesses in the skin. Sepsis in the mouth and teeth or after dental procedures or in association with infection of the respiratory or urogenital tract can also lead to septic arthritis. Direct penetrating trauma to the joint with sharp objects or from major traumatic injury can lead to joint infection as well. Joint aspiration or injection and surgical procedures such as joint replacement may also result in joint infection. Additionally, osteomyelitis often spreads to involve the joint. This is especially common in young children. Finally, infection of the soft tissues adjacent to the joint, such as inflamed bursae or tendon sheaths, can spread to involve the joint space. Spread of infection by the haematogenous route is still the most frequent cause of joint sepsis.

Symptoms of septic arthritis include malaise and fever, acute hot joint or joints together with acute inflammation: swelling and joint effusion, redness, pain and loss of function.

The most common causative organism of septic arthritis is *Staphylococcus aureus.* In neonatal septic arthritis, *Escherichia coli* and *Haemophilus influenzae* are also common pathogens. In children up to 5 years *Haemophilus influenzae* is the most common cause of haematogenous joint sepsis. Gram-negative intestinal bacteria are also common pathogens in the elderly and those with diabetes mellitus or prosthetic joints. In cases of penetrating injury, and in intravenous drug abusers, infection with *Pseudomonas aeruginosa* or *Staphylococcus epidermidis* are often found. In healthy young adults *Neisseria gonorrhoeae* or meningococcal infection are sometimes the cause of septic arthritis. Chronic low grade septic arthritis, especially in the spine, can be the result of infection with micro-organisms such as Mycobacteria or *Brucella abortus.* Furthermore, within acquired immune deficiency syndrome sufferers the range of joint pathogens is diverse.

Some of the most common septic arthritis pathogens include, but are not limited to, (1) Gram positive: *Staphylococcus aureus* (80% cases), *Streptococcus pyogenes*/*pneumoniae;* (2) Gram negative: *Haemophilus influenzae, Neisseria gonorrhoeae*/*meningitidis, Pseudomonas aeruginosa, Bacteroides fragilis, Brucella* species, *Salmonella* species, fusiform bacteria; (3) acid-fast bacilli: *Mycobacterium tuberculosis,* atypical mycobacteria; and (4) Spirochaetes: *Leptospira icterohaemorrhagica.*

Accordingly, the term "septic arthritis" as used herein and in the claims includes infections of the joint and surrounding tissues caused by the above listed pathogens as well as any other pathogens having the ability to infect the joint and surrounding tissues. Surrounding tissues include, but are not limited to, surrounding muscle, related tendons, connecting bones, bursae, tendon sheaths, synovium, synovial fluid, and related cartilage.

The term "treating septic arthritis" includes eradication of the pathogens/bacteria causing the underlying infection associated with septic arthritis, inhibition of bacterial growth, reduction in bacterial concentration, reduction in recovery time from infection, improvement, elimination, or reduction of symptoms of infection such as swelling, necrosis, fever, pain, weakness, and or other indicators as are selected as appropriate measures by those skilled in the art.

Tigecycline (formerly and often still referred to as "GAR-936") is a 9-t-butylglycylamido synthetic derivative of a new class of antibiotics called glycylcyclines. This new class of tetracycline derivatives has demonstrated excellent *in vitro* activity against a large number of gram positive and gram negative, aerobic and anaerobic organisms, including methicillin-resistant *Staphylococcus aureus* (MRSA), vancomycin-resistant enterococci (including *Enterococcus faecalis),* penicillin-resistant/macrolide-resistant pneumococci, *Prevotella* spp., peptostreptococci, and *Mycobacterium* spp. (Boucher et al., Antimicrob Agents Chemother. 2000; 44(8): 2225-2229, Gales et al., Antimicrob Agents Chemother. 2000; 46: 19-36, Goldstein et al., Antimicrob Agents Chemother. 2000; 44(10): 2747-2751). Tetracyclines are bacteriostatic agents, which act to inhibit bacterial protein synthesis. The glycylcyclines have been developed to overcome the bacterial mechanisms of resistance to tetracyclines, even though their exact mechanism of action has not yet been determined (Rasmussen et al., Antimicrob Agents Chemother 1995; 38: 1658-1660).

Tigecycline concentrates in bone, bone marrow, joint, and synovial fluid as well as many other organs and tissues of interest. Furthermore, it has been discovered that tigecycline concentrates in infected portions of the above described tissues. Studies of the pharmacokinetics of intravenous tigecycline in humans have shown that there is a rapid distribution phase, with a prolonged half-life (40 to 60 hours) and a high volume of distribution at steady state (7 to 14 L/kg). Animal studies with radiolabeled tigecycline suggest that this rapid distribution phase and high volume distribution at steady state represent penetration of tigecycline into tissues including lung and bone.

For example, the distribution of tigecycline in rat tissues has been shown in Sprague-Dawley rats when given [¹⁴C] tigecycline at a dosage of 3 mg/kg by 30-minute IV infusion. In general, radioactivity was well distributed to most tissues, with the highest overall exposure observed in bone. Exposure in tissues showing the highest concentrations were as follows: bone>bone marrow>salivary gland, thyroid, spleen, and kidney. In each of these tissues, the ratio of area under the concentration-time curve (AUC) in tissue to AUC in plasma was greater than 10. In this study, the ratio of AUC in the rat lung to AUC in the plasma was 4.4. Additionally, it has been demonstrated that intravenously administered tigecycline penetrates bone tissue in humans and intravenous administration extends concentration of tigecycline in synovial fluid in human over time.

The inventors have discovered that tigecycline is a useful treatment of osteomyelitis and septic arthritis. The antimicrobial spectrum is broad, including all the pathogens found in nosocomial bone and joint infections. The pharmacokinetic properties are favorable, since the drug may be administered twice daily. Moreover, bone penetration and drug levels above the minimum inhibitory concentration (MIC) were found in almost every sample collected. Minimum inhibitory concentration is a method of determining the efficacy of a compound in inhibiting bacterial growth. It is the lowest concentration of an antimicrobial agent that inhibits growth of a micro-organism and should correspond to concentrations required in sera of the mammal for the most minimal treatment. Additionally, tigecycline provided a good safety profile in humans, demonstrating that the antimicrobial should be suitable for clinical studies on orthopedic infections.

Accordingly, one aspect of the invention provides a method for treating infections of the bone, bone marrow, joint and surrounding tissue, and a method for treating osteomyelitis and/or septic arthritis in a mammal by administering to the mammal a pharmacologically effective amount of tigecycline. The bone, bone marrow, joint and surrounding tissue infections and osteomyelitis and/or septic arthritis and maybe caused by any of the commonly found pathogens, such as the pathogens discussed above, which include gram negative bacteria, gram positive bacteria, anaerobic bacteria and aerobic bacteria. For example, the infection may be comprised of, but not limited to, *Staphylococcus, Acinetobacter, Mycobacterium, Haemophilus, Salmonella, Streptococcus, Enterobacteriaceae, Enterococcus, Escherichia, Pseudomonas, Neisseria, Rickettsia, Pneumococci, Prevotella, Peptostreptococci, Bacteroides Legionella,* beta-haemolytic streptococci, and group B streptococcus. In preferred embodiments, the infection is comprised of *Neisseria, Mycobacterium, Staphylococcus,* and *Haemophilus.* In more preferred embodiments the infection is comprised *of Escherichia coli, Neisseria meningitidis, Neisseria gonorrhoeae, Mycobacterium tuberculosis, Staphylococcus aureus, Staphylococcus epidermidis, Streptococcus pyogenes, Streptococcus pneumoniae, Haemophilus influenzae, Enterococcus faecium, Rickettsia prowazekii, Rickettsia typhi, Rickettsia rickettsii, Mycobacterium leprae, Mcyobacterium abscessus,* or *Mycoplasma pneumoniae.*

In one embodiment of the present invention there is provided a method of treating infections of the bone, bone marrow, joint and surrounding tissue, and a method for treating osteomyelitis and/or septic arthritis caused by the bacterial strains (such as those described above) that demonstrate antibiotic-resistance by administering a pharmaceutically effective amount of tigecycline. For example, the exhibited resistance may be, but is not limited to, methicillin resistance, glycopeptide resistance, tetracycline resistance, oxytetracycline resistance, doxycycline resistance; chlortetracycline resistance, minocycline resistance, glycylcycline resistance, cephalosporin resistance, ciprofloxacin resistance, nitrofurantoin resistance, trimethoprim-sulfa resistance, piperacillin/tazobactam resistance, moxifloxacin, vancomycin resistance, teicoplanin resistance, penicillin resistance, and macrolide resistance.

In a preferred embodiment, the glycopeptide resistance is vancomycin resistance. In another preferred embodiment, the infection is comprised of *S. aureus* exhibiting resistance from either glycopeptide resistance, tetracycline resistance, minocycline resistance, methicillin resistance, vancomycin resistance or resistance to a glycylcycline antibiotic other than tigecycline.

In another preferred embodiment, the infection comprises *Acinetobacter baumannii* that may or may not exhibit antibiotic resistance such as cephalosporin resistance, ciprofloxacin resistance, nitrofurantoin resistance, trimethoprim-sulfa resistance, and piperacillin/tazobactam resistance. In another embodiment, the infection is comprised of *Mycobacterium abscessus* that may or may not exhibit moxifloxacin resistance.

In treatment of humans and other mammals, tigecycline is most commonly administered intravenously, although other administration paths are available to one of skill in the art. Doses of up to 100 mg administered during a one-hour infusion can be tolerated in human subjects. Twice-daily administrations over nine days of 75 mg or more in 200 ml infusions over one hour to subjects having been fed 30 minutes before infusion resulted in gastrointestinal intolerance in all subjects including nausea and vomiting. Twice-daily administration of 25-50 mg in 200 ml infusions over one hour was tolerated. A single infusion of 100 mg was also tolerated resulting in mean peak serum concentrations of 0.9 to 1.1 micrograms/ml.

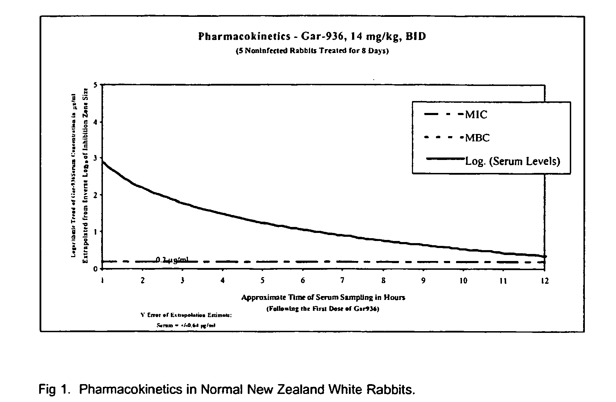

Administration of 14 mg/kg twice daily to New Zealand White Rabbits resulted in steady levels higher than the minimum inhibitory concentration. See Figure 1. The minimum inhibitory concentrations (MIC) and minimum bactericidal concentrations (MBC) for tigecycline for the MRSA strain used in this study were less than 0.2 µg/ml and 0.2 µg/ml, respectively. Measuring the MBC provides a method of determining the efficacy of a compound in killing bacteria. The MBC technique establishes the lowest level of a bactericidal agent that will kill at least 99.9% of the organisms in a standard inoculum.
MIC and MBC were determined by Mercier *et al.* for tigecycline against vancomycin resistant *E. faecium* to be 0.125 µg/ml and between 16 and 32 µg/ml, respectively. For *S. aureus,* minimum inhibitory concentrations and minimum bactericidal concentrations were between 0.25 and 1 µg/ml and 16 and 64 µg/ml, respectively. In a compassionate use study, the inventors found the minimum inhibitory concentration of tigecycline against *M. abcessus* in a human patient to be 0.25 µg/ml.

In mammals, methicillin-resistant *S. aureus* may be treated with tigecycline in the range of 5 mg/kg to 60 mg/kg twice daily, more preferably 10 mg/kg to 40 mg/kg, more preferably 12 mg/kg to 20 mg/kg. Appropriate dosages for treatment of other pathogens will be apparent to one of skill in the art.

In a compassionate use study, one human patient suffered from spina bifada with resultant paraplegia. The patient was severely allergic to sulfa drugs and presented with methicillin-resistant bacteremia from infected heel decubitis. The patient also had skin breakdown over the right ischium. The ulcer was debrided, but it did not heal. An MRI revealed osteomyelitis and a section of the bone was positive for infection from *Acinetobacter baumannii.*

The *A. baumanii* was resistant to cephalosporins, ciprofloxacin, nitrofurantoin, and demonstrated intermediate resistance to trimethoprim-sulfa and piperacillin/tazobactam. The organism was susceptible to imipenem, gentamicin, and tobramycin. The patient was treated with meropenem and tobramycin. Meropenem was later replaced with aztreonam due to eosinophilia. Aztreonam was later discontinued because of persistent eosinophilia. Tobramycin was also discontinued because of increased creatinine. The patient was then treated with tigecycline for two months with either 50 mg every 12 hours or 50 mg every 24 hours. Within one month of receiving treatment with tigecycline, an MRI showed resolution of the osteomyelitis and marked improvement was seen in fluid collected from right ischial area. The patient was reported doing well ten weeks post treatment with tigecycline.

In another compassionate use study, a patient with anhydrotic ectodermal dysplasia with immunodeficiency had a three and one-half year history of vertebral osteomyelitis with a *Mycobacterium abscessus* infection. Debridement was accomplished after one year of infection with placement of hardware. The patient showed some improvement with cefoxitan, clarithromycin, and amikacin. The amikacin was later stopped due to renal damage. Linezolid and azithromycin were later added to the treatment regimen. The organism was determined to be resistant to moxifloxacin.

The patient presented later with a new vertebral osteomyelitis just above the site of the old infection. A biopsy was performed and it was determined that no additional debridement was needed. The organism was found to be sensitive only to cefoxitin. It was determined that an additional antimicrobial agent would be helpful and the organism was found to be susceptible to tigecycline. Tigecycline was administered up to MIC 0.25 micrograms/ml. The patient's white blood cell count was normal while hypogammaglobulinemia was present and lymphocytic function decreased. The patient had also been under treatment with IL-12, but IL-12 was held during antibiotic treatment. The patient was reported to be doing well a year after the treatment.

Another embodiment of the present invention provides a method of treating infections of the bone, bone marrow, joint and surrounding tissue, and a method for treating osteomyelitis and/or septic arthritis in a mammal, preferably a human, comprising administering to the mammal a pharmacologically effective amount of tigecycline and an antimicrobial agent from the ansamycin family, which includes the rifamycin and the streptovaricin groups of antibiotics. The rifamycin family includes rifampin, rifapentine, rifaximin, and preferably, rifampin. These macrocyclic antibiotics have bactericidal activity because of their propensity for binding to RNA polymerase. These antibiotics are useful in combination with tigecycline because they effect different steps in bacterial protein synthesis. While the rifamycins effect the activity of RNA polymerase and limit production of messenger RNA, tigecycline effects the activity of ribosomes and the production of proteins from the messenger RNA. The mode of action of tigecycline appears to be related to inactivation of the 70S ribosomes through binding to a tetracycline-binding site in the 30S ribosomal subunit with a somewhat different orientation than does tetracycline. (Bauers et al., J. Antimicrob Chemother. 2004; 53(4): 592-599).

The present inventors have discovered that tigecycline in combination with an antibiotic of the rifamycin class of antimicrobials provides additive antimicrobial effect in infected tissue. In an investigation with rabbits inoculated at the tibia with methicillin-resistant *S. aureus,* treatment of osteomyelitis with tigecycline in combination with rifampin demonstrated no infection in bone in 10 rabbits while controls showed infection in 11 of 15 rabbits. Treatment in bone marrow also demonstrated no infection in 10 rabbits while controls showed 5 infected rabbits of 15 rabbits tested. Furthermore, treatment of osteomyelitis in rabbits with tigecycline alone demonstrated infection in the bone of one rabbit of 10 and no infection in the marrow.

In mammals, rifampin treatment may be in the range of 10 mg/kg to 100 mg/kg twice daily, more preferable it may be in the range of 20 mg/kg to 70 mg/kg twice daily, more preferably it may be in the range of 30 mg/kg to 50 mg/kg twice daily. In New Zealand White rabbits infected with MRSA, treatment of 40 mg/kg resulted in bactericidal activity. The minimum inhibitory concentration and minimum bactericidal concentration levels for rifampin against the MRSA strain were 0.78 µg/ml and 1.56 µg/ml, respectively, yielding a ratio of 0.5.

Human oral administration of rifampin is available with capsules of 150 and 300 mg. Following a single 600 mg dose in healthy human adults, peak serum concentrations averaged 7 micrograms/ml but with wide variance from 4 to 32 micrograms/ml. Administration of 600 mg intravenously to healthy human adults over 30 minutes resulted in mean peak serum concentrations of about 17 micrograms/ml.

Administration of tigecycline is preferably administered intravenously or intramuscularly, while rifampin may be administered intravenously, intramuscularly, orally or by other means of administration known to the art such as transbuccal, intrapulmonary or transdermal delivery systems. Co-administration may include a combination of any of these methods. For example, tigecycline may be administered intravenously while rifampin may be administered orally. Co-administration includes simultaneous or sequential administration, in any order and does not necessarily imply administration at the same time or same day or same time course schedule. Preferably, concentrations of both tigecycline and rifampin are concurrently maintained well above the minimum inhibitory concentration.

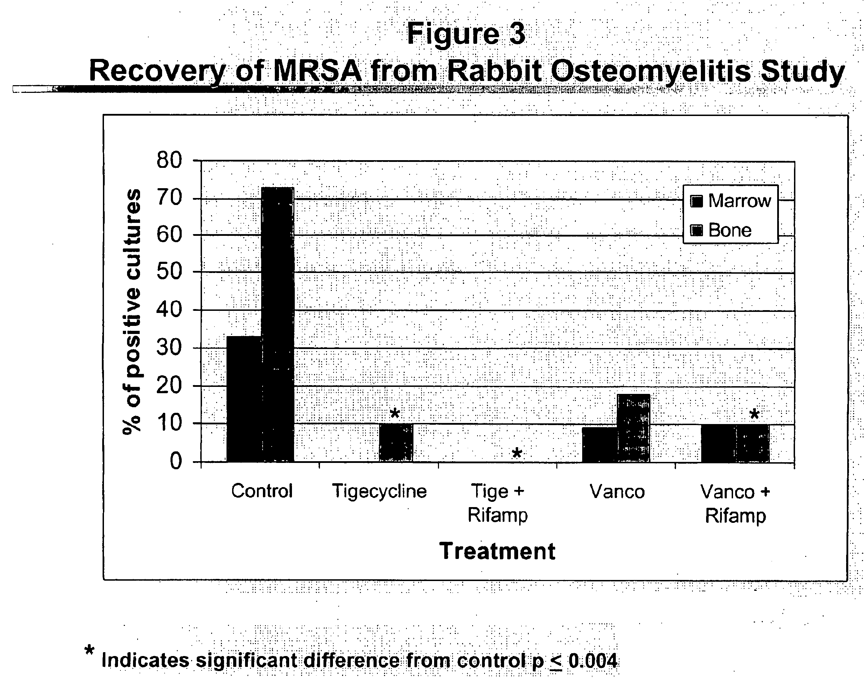

In a trial by the inventors, a group of rabbits infected with methicillin-resistant *S*. *aureus* and treated with tigecycline showed lower colony forming units in bone and marrow than the infected, untreated control group or the group treated with vancomycin at the end of the treatment period. The MIC and MBC for tigecycline (0.2 µg/ml) were lower than that of vancomycin (0.39 µg/ml and 0.78 µg/ml), which is more conducive to the resolution of osteomyelitic infections. The association of tigecycline and rifampin allowed the complete eradication of bacteria from the bone and marrow, whereas in the vancomycin plus rifampin group a sample was still positive. *See* Figure 3. Treatment was successful with subcutaneous administration of 14 mg/kg of tigecycline twice daily and oral administration of 40 mg/kg of rifampin twice daily. These data demonstrate that osteomyelitis in rabbits with methicillin-resistant S. *aureus* infection is effectively treated with a combination of tigecycline and rifampin.

Accordingly, given the disclosure presented herein, such as the dose and treatment regimens (i.e. length and mode of administration, and time course of therapy) used in the above described compassionate use studies, typical dose and treatment regimens of common antibiotics administered to patients to treat infections with the listed pathogens, and dose and treatment regimens used in the rabbit study, one skilled in the art would appreciate the appropriate dose and treatment regimen to administer to a mammal to achieve a pharmacologically effective amount of tigecycline and/or additional antimicrobrials such as rifampin, to treat osteomyelitis and/or septic arthritis. One skilled in the art would appreciate that factors such as the extent of the infection, overall health, weight, and age of the patient would effect the desired dose and treatment regiment.

The term "pharmacologically effective amount" means, consistent with considerations known in the art, the amount of antimicrobial agent effective to achieve a pharmacologic effect or therapeutic improvement without undue adverse side effects, including but not limited to, inhibition of bacterial growth, reduction in bacterial concentration, reduction in recovery time from infection, improvement, elimination, or reduction of symptoms of infection or other disease such as swelling, necrosis, fever, pain, weakness, and or other indicators as are selected as appropriate measures by those skilled in the art.

Another embodiment of the present invention provides the use of tigecycline with or without an antimicrobial agent selected from the group consisting of rifamycin, rifampin, rifapentine, rifaximin, or streptovaricin (preferably rifampin) for the manufacture of a medicament for treatment infections of the bone, bone marrow, joint and surrounding tissue, and osteomyelitis and/or septic arthritis in a mammal, preferably a human.

Another embodiment provides a pharmaceutical composition for the treatment of infections of the bone, bone marrow, joint and surrounding tissue, and osteomyelitis and/or septic arthritis in a mammal, preferably a human, comprising tigecycline, with or without an antimicrobial agent selected from the group consisting of rifamycin, rifampin, rifapentine, rifaximin, or streptovaricin (preferably rifampin), and pharmaceutically acceptable diluents, preservatives, solubilizers, emulsifiers, adjuvants and/or carriers conventionally used in pharmaceutical and veterinary formulations. The present pharmaceutical formulations may be adapted for administration to humans and/or animals.

Another embodiment of the present invention provides the use of tigecycline with or without an antimicrobial agent selected from the group consisting ofrifamycin, rifampin, rifapentine, rifaximin, or streptovaricin (preferably rifampin) for manufacture of a medicament for treatment of infections of the bone, bone marrow, joint and surrounding tissue, and osteomyelitis and/or septic arthritis in a mammal, preferably a human.

It is to be understood that in the various embodiments of the present invention, tigecycline and/or rifampin or other antimicrobials may by present as pharmaceutically acceptable salts thereof. For example, such salts may include but are not limited to the hydrochloride, sulfate or phosphate salts. They may also include the acetate, citrate or lactate salts, for example.

The medicament or pharmaceutical composition is administered at a dose to achieve a pharmacologically effective amount of the tigecycline and a pharmacologically effective amount of an antimicrobial agent selected from the group consisting of rifamycin, rifampin, rifapentine, rifaximin, or streptovaricin (preferably rifampin). The pharmaceutical composition and/or medicament further comprise pharmaceutically acceptable diluents, preservatives, solubilizers, emulsifiers, adjuvants and/or carriers. These may include but are not limited to, saccharose, mannitol, sorbitol, lecithins, polyvinylpyrrolidones, microcrystalline celluloses, methylcelluloses, carboxymethylcelluloses, hydroxyethylcelluloses, hydroxypropyl celluloses; starches, polyacrylates, ethylcelluloses, hydroxypropyl cellulose, hydroxypropylmethylcellulose and their derivations, triacetin, dibutylphthalate, dibutylsebacate, citric acid esters, polyethyleneglycols, polypropyleneglycols, polyvinylpyrrolidone, lactose, sucrose, magnesium stearate, talc, or silicone oil.

For oral administration, the pharmaceutical formulations may be utilized as, for example, tablets, capsules, emulsions, solutions, syrups or suspensions. For parenteral administration, the formulations may be utilized as ampoules, or otherwise as suspensions, solutions or emulsions in aqueous or oily vehicles. The need for suspending, stabilizing and/or dispersing agents will of course take into account the solubility of the active compounds in the vehicles which are used in particular embodiments. The formulations may additionally contain physiologically compatible preservatives and antioxidants.

The pharmaceutical formulations may also be utilized as suppositories with conventional suppository bases such as cocoa butter or other glycerides. Alternatively, the formulations may be made available in a depot form that will release the active composition slowly in the body, over a pre-selected time period.

### EXAMPLES

### EXAMPLE 1: Treatment of osteomyelitis in rabbits with tigecycline

This example shows the treatment of osteomyelitis in rabbits with tigecycline and tigecycline in combination with rifampin. Comparison studies with vancomycin and the combination of vancomycin with rifampin were also performed. Data demonstrate improved antimicrobial efficacy with tigecycline over vancomycin, and with tigecycline in combination with rifampin over vancomycin in combination with rifampin. Additionally, tigecycline in combination with rifampin provided complete protection against methicillin-resistant *S. aureus* within its test group.

### Generation of Standard Curves for Diffusion Bioassays

Normal NZW rabbit serum (Fisher Scientific) and normal, uninfected rabbit tibia bone were used to generate standard curves for tigecycline (Wyeth-Ayerst Research, Pearl River, New York), vancomycin (Abbott Laboratories, Chicago, Illinois), and rifampin (Merrell Pharmaceuticals Inc. Kansas, Missouri). Bioassays were performed with each drug to generate the standard curves for antibiotic concentration in serum and/or tibial bone.

The organism used for the bioassay was *Bacillus cereus* ATCC11778. Serum standards were prepared using two-fold serial dilutions with either antibiotic to yield concentrations of 25 µg/ml to 0.20 µg/ml drug in Normal NZW rabbit serum. Bone eluate standards were prepared for tigecycline by thoroughly cleaning noninfected rabbit tibias with 70% ethanol in a sterilized fume hood. Each tibia was broken into small chips of approximately 0.5 cm² using a grinder. The chips were placed into a sterile, 50 ml conical centrifuge tube and weighed. One milliliter of sterile, 0.9% normal saline was added for each gram of bone chips. The solution was thoroughly vortexed for two minutes. The resulting bone eluate was allowed to shake at 180 rpm in a cold room at 4°C, for 12 hours. The samples were centrifuged at 4000 rpm for 3 minutes prior to assay, to pellet the chips.

The diameter of the zone of growth inhibition around each well was measured, in millimeters. A standard curve was generated for tigecycline concentration in both serum and bone eluate and for vancomycin in serum by plotting the known antibiotic concentration against its resulting zone of inhibition measurement.

### Pharmacokinetics of Tigecycline

A baseline group of 6 uninfected rabbits were subcutaneously administered 14 mg/kg tigecycline, reconstituted in sterile water, every 12 hours, for a period of 8 days. Blood samples were drawn at the following approximate intervals, post-initial antibiotic treatment: 1 hour, 3 hours, 6 hours, 12 hours, 171 hours and 180 hours (time of sacrifice). One-half milliliter of blood was collected with standard techniques. Samples were immediately placed into sterile, 1.5 ml centrifuge tubes. Following euthanasia, both tibias were thoroughly cleansed with 70% ethanol and then harvested, after removal of all soft tissue. Tibias were placed into separate, sterile 50 ml centrifuge tubes and stored at -70°C.

Serum samples were stored at -70°C until the bioassay was performed. Bone samples were prepared as previously described. Seeded agar plates were prepared and samples were loaded in triplicate, to the seeded plates and incubated at 30°C for 18 hours. The diameter of the zone of growth inhibition around each well was measured and concentrations of tigecycline were extrapolated from the standard curve.

### Minimum Inhibitory Concentration and Minimum Bactericidal Concentration Determinations

The minimum inhibitory concentrations (MIC) of tigecycline, vancomycin and rifampin were determined using an antibiotic two-fold tube-dilution method. The minimum bactericidal concentrations were also determined. The limits of sensitivity of this method were 25 µg/ml to 0.20 µg/ml.

### Induction of Tibial Osteomyelitis

A localized *S*. *aureus* osteomyelitis was percutaneously induced in the left lateral tibial metaphysis of all rabbits within all six study groups. The strain of methicillin-resistant *S. aureus* was obtained from a patient with osteomyelitis undergoing treatment.

*Preparation of the Infective Media: S. aureus* was incubated overnight in Mueller Hinton Broth(Difco Laboratories, Detroit, Michigan) medium spiked with 40 µg/ml oxacillin, at 37°C. The bacterial concentration of the culture was adjusted to 10⁷ CFU's/ml.

*Rabbit Infection Procedure:* New Zealand white rabbits (Ray Nicholl's Rabbitry, Lumberton, Texas), eight to 12 weeks old and weighing 2.0 to 3.5 kg, were utilized for the study. After anesthesia was given, an 18-gauge needle was inserted percutaneously through the lateral aspect of the left tibial metaphysis into the intramedullary cavity. Next, 0.15 ml of 5% sodium morrhuate (American Regent Laboratories, Inc., Shirley, New York), 0.1 ml of *S. aureus* (10⁷ CFU/ml), and 0.2 ml of sterile, normal saline, 0.9%, were injected sequentially. The infection was allowed to progress for 2 weeks, at which time the severity of osteomyelitis was determined radiographically (Table 1).

*Treatment Groups:* At the end of two weeks, post infection, the rabbits with localized proximal tibial osteomyelitis (confirmed radiographically as Grades 2-4) were separated into six study groups. Group 1 (control group): infected but left untreated for the duration of the study. Group 2: rabbits were treated for 4 weeks with subcutaneous vancomycin at 30 mg/kg twice daily. Group 3: rabbits were treated for 4 weeks with subcutaneous vancomycin at 30 mg/kg twice daily plus oral rifampin at 40 mg/kg twice daily in 0.5% methylcellulose. Group 4: rabbits were treated for 4 weeks with subcutaneous tigecycline at 14 mg/kg twice daily. Group 5: rabbits were treated for 4 weeks with subcutaneous tigecycline at the same dose as in the rabbits in Group 4, plus oral rifampin at 40 mg/kg twice daily in 0.5% methylcellulose. Rabbits receiving oral rifampin (Groups 3 and 5) were given an oral nutritional supplement (Ensure Plus^{®}, Abbott Laboratories, Columbus, Ohio) and a *Lactobacillus spp.* preparation (Kvvet Supply, 3190 NRoad, David City, Nebraska) daily. Group 6: rabbits were treated for 1 week with subcutaneous tigecycline at the same dose as in Group 4, but were sacrificed 3 hours after administration of the last dose. At that time, blood and infected bone samples were collected and tigecycline concentration was determined. Groups 1 to 5 were left untreated for 2 weeks after treatment phase of the experiment and sacrificed at 8 weeks after infection.

### Radiographic Assessment

Radiographs of bilateral tibias were taken at initiation of therapy (2 weeks after infection), at the end of antibiotic therapy (6 weeks after infection), and at sacrifice (8 weeks after infection). Radiographs were scored according to a visual scale (Table 1) by three investigators, each blinded to the treatment group, and the grades were averaged.

**TABLE 1**

| Criteria for Radiographic Osteomyelitis Severity Grading in Rabbits | |
|---|---|
| **Grade*** | **Description of Changes** |
| 0 | Normal, no change compared with right tibia |
| 1+ | Elevation or disruption of periosteum, or both; soft tissue swelling |
| 2+ | < 10% disruption of normal bone architecture |
| 3+ | 10 - 40% disruption of normal bone architecture |
| 4+ | > 40% disruption of normal bone architecture |

* Visually estimated percentage of disrupted bone.

### Determination of Serum Levels of Antibiotic

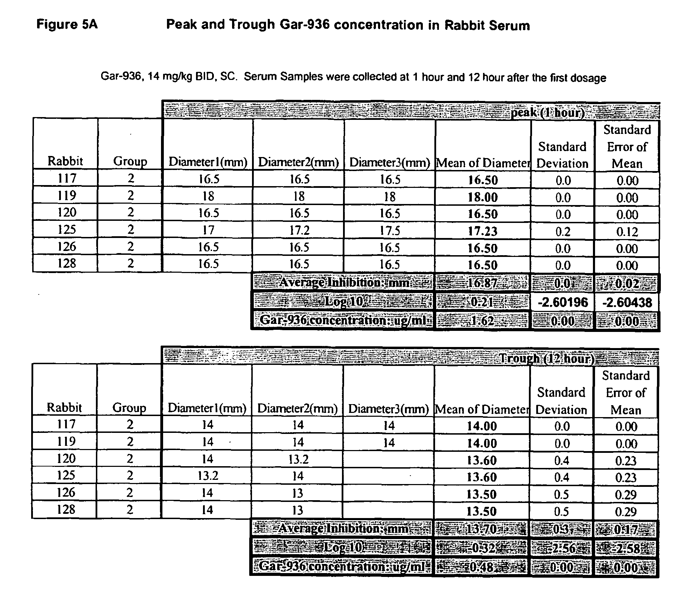

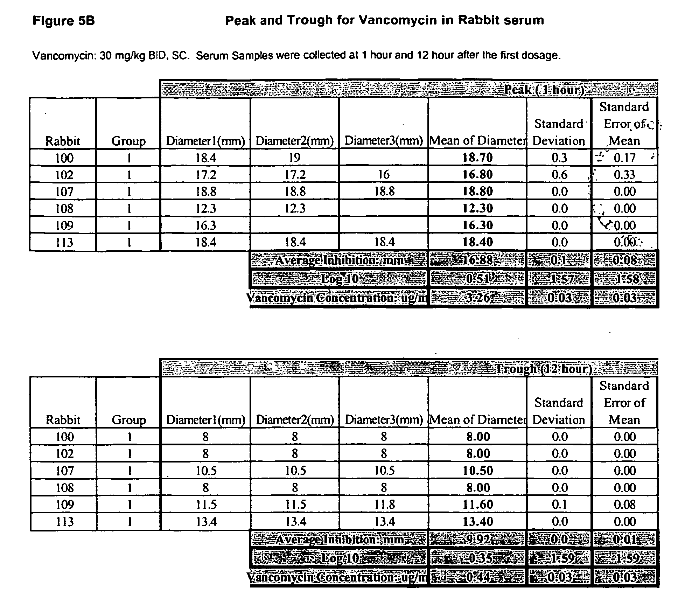

Peak and trough levels of antibiotic were determined for Groups 2 and 4 at 1 hour (peak) and 12 hour (trough) after the initial antibiotic administration. See Figures 5A and 5B. Antibiotic concentrations were determined by means of a bioassay. Antibiotic diffusion assay was performed as described above. Concentrations of antibiotic were extrapolated from the respective standard curves.

### Determination of Bacterial Concentration per Gram of Bone and Bone Marrow

After sacrifice, gross cultures were performed for right and left tibias. Quantitative counts of *S*. *aureus,* in CFUs per gram, of left tibial bone and marrow were determined for all study groups.

*Culture Preparation:* The bone marrow and the intramedullary canal of bilateral tibias were swabbed with sterile cotton tip applicators for gross cultures analysis of left tibias and quality assurance checks of right tibias. The inoculated applicator was streaked onto blood plates and then placed into 5 ml of sterile TSB. The plates and tubes were then incubated at 37°C for 24 hours and growth and/or turbidity was recorded.

The bone marrow was placed into a sterile, 50 ml centrifuge tube and weighed. The bone fragments were broken into 0.5 cm² chips, placed into a sterile, 50 ml centrifuge tube, and the final product weighed. Normal sterile saline, 0.9%, was added in a 3 to 1 ratio (3 ml saline/gram of bone or marrow) and the suspensions were vortexed for 2 minutes. Six ten-fold dilutions of each suspension were prepared with sterile, normal saline, 0.9%. Twenty-microliter samples of each dilution, including the initial suspension, was plated, in triplicate, onto blood agar plates and incubated at 37°C for 24 hours. CFUs were counted at the greatest dilution for each tibia sample. The *S. aureus* concentration was calculated in CFUs per gram of bone or bone marrow. The calculated resultant was multiplied by 3 for bone samples and by 4 for bone marrow, in order to account for their initial dilutions in saline and for the adsorption of marrow into the saline. The mean log of the *S*. *aureus* concentration for each was calculated.

### Statistical Analysis of Experimental Data

The standard deviation and standard error of the mean were calculated for all raw data, including disc diffusion measurements, weight variances, radiograph grades, and bacterial counts. Linear regression analysis, least squares method, were performed for the antibiotic diffusion standard curves using the base ten log of the antibiotic concentrations to plot the concentration (in µg/ml) versus the zone of inhibition measured (in millimeters). All subsequent diffusion measurements were extrapolated to micrograms/milliliter of antibiotic concentration from the standard curve utilizing the slope and y-intercept values derived from the least squares calculations.

### Minimum Inhibitory Concentrations and Minimum Bactericidal Concentrations

For the strain of methicillin-resistant *S*. *aureus* (inoculum of 10⁶ CFU/ml) used in the study, the minimum inhibitory concentrations and minimum bactericidal concentrations for tigecycline were less than 0.2 µg/ml and 0.2 µg/ml, respectively. The minimum inhibitory concentration and minimum bactericidal concentration levels for vancomycin were 0.39 µg/ml and 0.78 µg/ml, respectively, yielding MIC/MBC ratio of 0.5. The minimum inhibitory concentration and minimum bactericidal concentrations levels for rifampin were 0.78 µg/ml and 1.56 µg/ml, respectively, yielding a ratio of 0.5.

### Drug Kinetic Levels in Bone and Serum

All concentrations of antibiotic were derived from the respective standard curves. The logarithmic trends of the concentrations of tigecycline (14 mg/kg twice daily) in the sera of uninfected animals group are shown in Figure 1. The tigecycline, as depicted in Figure 1, eliminated slowly, maintaining a steady level higher than MIC (0.2 µg/ml) by 12 hour (trough). Peaks and troughs of tigecycline (14 mg/kg twice daily) and vancomycin (30 mg/kg twice daily) in the serum of infected rabbits after administration of the respective drugs are shown in Figures 5a and 5b. The bone concentrations of tigecycline (14 mg/kg, Bid) in the infected rabbits group were measured separately in the infected tibia at the end of treatment, in which they averaged 0.78 µg/ml +/- 0.01 µg/ml, and in the uninfected tibia, in which they averaged 0.49 µg/ml +/- 0.01 µg/ml. The difference was statistically significant (p < 0.05).

### Radiographic Findings

A stage 2 to 4 osteomyelitis, according to Table 1, was induced in all the infected animals. The initial radiographic grades were similar between the groups. The average grades for tigecycline, tigecycline + rifampin and vancomycin + rifampin groups at t=14 days were significantly greater than the average grades at t=56 days (p<0.05). The control group showed the least amount of improvement radiographically (0.2 +/- 0.2 or 9.1%), when compared with vancomycin (0.5 +/- 0.2 or 25%), with tigecycline (0.9 +/- 0.1 or 40.9%), with vancomycin + rifampin (0.9 +/- 0.1 or 40.9%) or with tigecycline + rifampin (0.8 +/- 0.1 or 40.0%) groups.

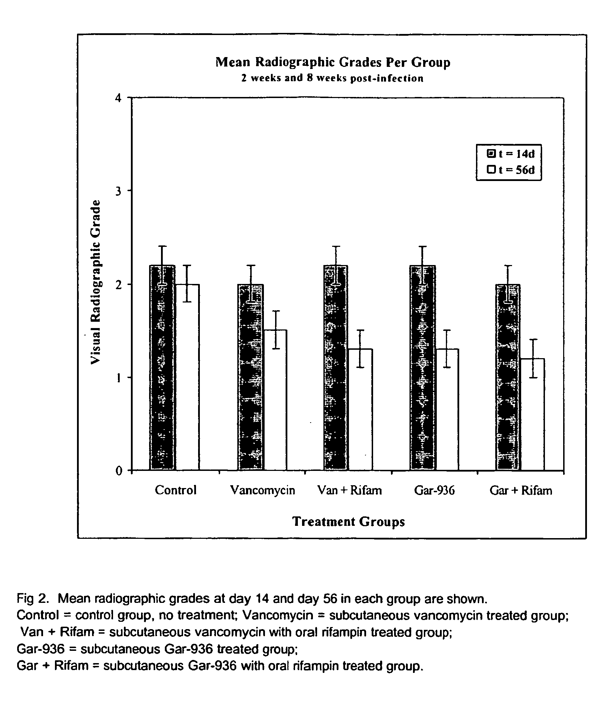

Figure 2 depicts the average radiographic severity for each group at t=14 and t=56 days. At the end of the study (t=56 days), average radiographic grades were compared between different groups. The average grades for tigecycline group, tigecycline + rifampin group and vancomycin + rifampin group at t=56 days were significantly lower than the average grades for the control group at t=56 days (p < 0.05). The key for figure 2 is as follows: Control = control group, no treatment; Vancomycin = subcutaneous vancomycin treated group; Van + Rifam = subcutaneous vancomycin with oral rifampin treated group; Gar-936 = subcutaneous Gar-936 treated group; Gar + Rifam = subcutaneous Gar-936 with oral rifampin treated group.

### Bone Cultures

A high percentage of tibias from untreated infected controls (n=15) revealed positive cultures (80%) for methicillin-resistant *Staphylococcus aureus* at a mean concentration of 9.21 x 10⁴ CFU/g bone. When compared to untreated controls, the vancomycin group (n=11), tigecycline group and tigecycline + rifampin group all demonstrated a significantly lower percentage of positive methicillin-resistant *Staphylococcus aureus* infection. In the vancomycin group, or 2 out of 11 samples (18.2%) were positive for MRSA, and the average bacterial concentration of the group was 1.4 x 10² CFU/ gram bone (p < 0.05). In the tigecycline group, 1 out of 10 samples was positive for methicillin-resistant *Staphylococcus aureus* and the average bacterial concentration in the group was 20 CFU/ gram bone, which is lower than either the controls or the vancomycin group (p < 0.05). One rabbit in vancomycin + rifampin group showed higher bacteria concentration than the control. The rabbits receiving tigecycline + rifampin treatment group demonstrated complete eradication of bacteria from the tibia (0.0 CFU/ gram bone in all the samples). Figure 3 compares the CFU/gram marrow and bone between all groups. Figure 3 demonstrates that tigecycline and tigecycline in combination with rifampin were an effective treatment for infection of the bone and infection of the marrow with respect to controls.

The key for figure 3 is as follows: Control = control group, no treatment; Vancomycin = subcutaneous vancomycin treated group; Gar-936 = subcutaneous Gar-936 treated group; Vancomycin + Rifampin = subcutaneous vancomycin with oral rifampin treated group; Gar-936 + Rifampin = subcutaneous Gar-936 with oral rifampin treated group.

### Adverse Events

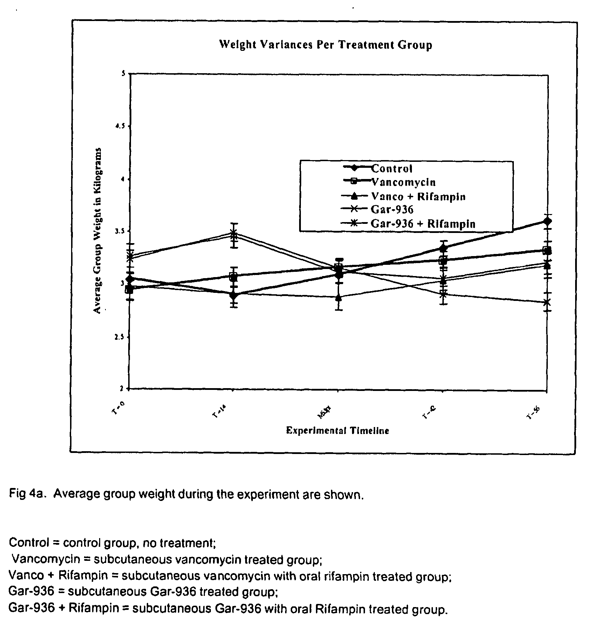

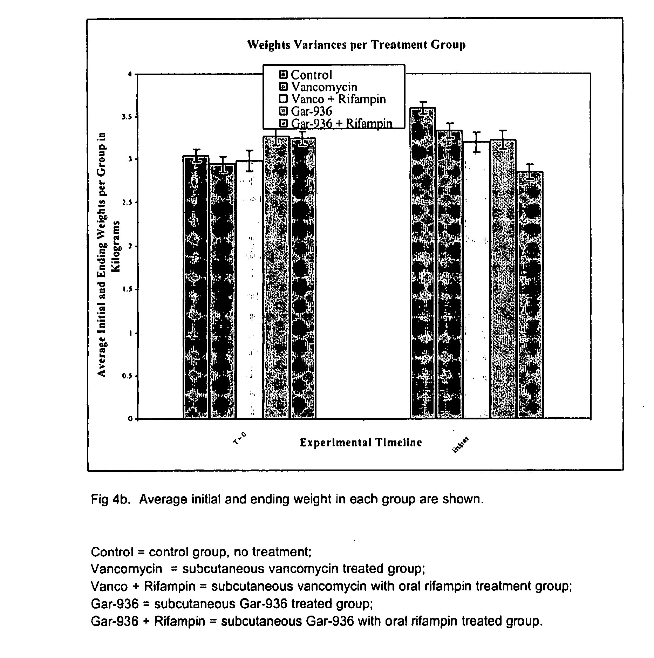

Of the 66 infected rabbits, a total of 6 died before completion of treatment. Of the 5 rabbits that died in the tigecycline treatment group, one of them was euthanized at day 19 because of severe impairment of nutritional status. Another rabbit died at the day 17 of tigecycline treatment due to gastroenterocolitis. Three of the rabbits in this group died at day 28 due to gastroenterocolitis and intolerance to anesthesia. One rabbit in the tigecycline + rifampin group died during treatment at day 15 due to gastroenterocolitis. The gastroenterocolitis was most likely caused by alteration of the normal flora of the large intestine. All rabbits were monitored weekly for weight variance. The control group showed the greatest mean gain (0.58 kg +/- 0.27), vancomycin the second greatest (0.39 kg +/- 0.26), vancomycin + rifampin group the third (0.21 kg +/- 0.32). Tigecycline group (-0.05 kg +/-0.32) and tigecycline + rifampin (-0.39 +/- 0.31) group both lost weight after the antibiotic treatment. Nearly all rabbits in the tigecycline group and tigecycline + rifampin group presented with mild to severe symptoms of gastric dysfunction approximately 1.0-1.5 weeks post-antibiotic initiation, including decreased appetite, dehydration, diarrhea, and/or weight loss. Figure 4A and B show the weight variances between all the groups. The key for figures 4A and B is as follows: Control = control group, no treatment; Vancomycin = subcutaneous vancomycin treated group; Vanco + Rifampin = subcutaneous vancomycin with oral rifampin treated group; Gar-936 = subcutaneous Gar-936 treated group; Gar-936 + Rifampin = subcutaneous Gar-936 with oral Rifampin treated group.

As for the safety, a higher number of deaths and side effects were seen in the groups of rabbits treated with tigecycline. Enterocolitis due to tigecycline may be caused by an extensive destruction of the normal microbial flora of the bowel. The symptoms were attenuated by the administration of oral probiotics. The broad antimicrobial spectrum of tigecycline, in contrast with the narrower spectrum of vancomycin, may help explain the difference observed between the treatment groups.

### Results

The count data for each animal in each tissue are listed in Table 2. The counts in the table are averages of triplicate measurements made on each tissue. Inspection of the data in Table 2 reveals that in treatment groups treated with test articles, the counts in most or all animals were 0. In the control group, non-zero counts were measured in marrow from 5 of 15 animals and in bone from 11 of 15 animals. There was considerable variation in the magnitude of the non-zero counts in the control groups, especially for bone.

The number of positive and negative cultures in each treatment group, and the p-values resulting from comparisons to control were was follows:

**TABLE 2**

| **Count of Colony Forming Units Per Gram of Bone and Marrow from Osteomyelitis Study In Rabbits** | | |
|---|---|---|
| Treatment Group | Counts (CFU/gm) Marrow | Counts (CFU/gm) Bone |
| Control 1 | 0 | 238 |
| 2 | 0 | 0 |
| 3 | 83,000,000 | 97 |
| 4 | 0 | 0 |
| 5 | 0 | 5000 |
| 6 | 0 | 2380 |
| 7 | 610,000 | 100000 |
| 8 | 22,000 | 1000 |
| 9 | 0 | 0 |
| 10 | 0 | 50 |
| 11 | 1700 | 1,100,000 |
| 12 | 0 | 0 |
| 13 | 4400 | 10,8000 |
| 14 | 0 | 72.9 |
| 15 | 0 | 106.3 |
| Tigecycline 1 | 0 | 178.6 |
| 2 | 0 | 0 |
| 3 | 0 | 0 |
| 4 | 0 | 0 |
| 5 | 0 | 0 |
| 6 | 0 | 0 |
| 7 | 0 | 0 |
| 8 | 0 | 0 |
| 9 | 0 | 0 |
| 10 | 0 | 0 |
| Tigecycline + | 0 | 0 |
| 2 | 0 | 0 |
| 3 | 0 | 0 |
| 4 | 0 | 0 |
| 5 | 0 | 0 |
| 6 | 0 | 0 |
| 7 | 0 | 0 |
| 8 | 0 | 0 |
| 9 | 0 | 0 |
| 10 | 0 | 0 |
| Vancomycin 1 | 1250 | 270.3 |
| 2 | 0 | 1315.8 |
| 3 | 0 | 0 |
| 4 | 0 | 0 |
| 5 | 0 | 0 |
| 6 | 0 | 0 |
| 7 | 0 | 0 |
| 8 | 0 | 0 |
| 4 | 0 | 0 |
| 5 | 0 | 0 |
| 6 | 0 | 0 |
| 7 | 0 | 0 |
| 8 | 0 | 0 |
| 9 | 0 | 0 |
| 10 | 0 | 0 |
| 11 | 0 | 0 |
| Vancomycin + | 0 | 0 |
| 2 | 530,000,000 | 1,040,000 |
| 3 | 0 | 0 |
| 4 | 0 | 0 |
| 5 | 0 | 0 |
| 6 | 0 | 0 |
| 7 | 0 | 0 |
| 8 | 0 | 0 |
| 9 | 0 | 0 |
| 10 | 0 | 0 |

### Data from Rabbit osteomyelitis comparison of tigecycline, vancomycin, and rifampin

In marrow, the proportion of positive cultures in the tigecycline and tigecycline + rifampin treatment groups was 0, which in comparison to the proportion of 0.33 (5/15) in the control group was almost statistically significant (p=0.06) at the conventional p=0.05 level. The proportions in the vancomycin and vancomycin + rifampin groups were not statistically significantly different from the control group. In bone, the proportion of positive cultures in each of the groups treated with test articles was statistically significantly lower than the proportion in the control group.

In an animal model of methicillin-resistant *Staphylococcus aureus,* endocarditis, 14 mg\kg bid tigecycline was shown to be more effective than 40 mg\kg vancomycin (Murphy, Antimicrob Agents Chemother 2000; 44(11): 3022-3027). In a rat model, dosages as high as 80 mg\kg\day were administered. However, in the rabbit model used herein, the administration of dosages higher than 14 mg/kg per day caused relevant morbidity and mortality in the animals (data not shown). Therefore, the above cited dosage was used in this study. Even though the goal was not to study the pharmacokinetics of tigecycline in rabbits, some drug levels measurements were performed in order to ensure that an adequate dosage was being used in the animal model. The data confirm that drug levels in serum were still above the MIC of the staphylococcal strain used 12 hours after the last administration. Moreover, the drug has displayed a relevant bone penetration, and therapeutic levels of tigecycline have been found in the infected and uninfected bone. The higher concentration of drug found in the infected bone is another relevant finding, which requires further study.

### EXAMPLE 2: Distribution of Tigecycline in Human Tissue after One Intravenous Administration of 100 mg.

This example shows the penetration of selected tissues in human subjects after a single intravenous administration of tigecycline. The data demonstrate a rapid distribution phase, with a prolonged half-life and a high volume of distribution at steady state. They further establish the penetration of bone, synovial fluid, lung, gall bladder, and colon in human subjects. Penetration improves treatment of bone and joint infections.

Studies of the pharmacokinetics of intravenous tigecycline in humans have shown that there is a rapid distribution phase, with a prolonged half-life (40 to 60 hours) and a high volume of distribution at steady state. Animal studies with radiolabeled tigecycline suggest that this rapid distribution phase and high volume of distribution at steady state represent penetration of tigecycline into tissues including lung and bone. Sprague-Dawley rats (18 males) were given carbon-14 tigecycline at a dosage of 3 mg/kg by 30-minute infusion. Concentrations or radioactivity were determined in tissues of 3 rats/time point at the end of the infusion and at 1, 8, 24, 72, and 168 hours after the end of infusion. For all tissues, peak radioactivity concentration were observed at the end of infusion. In general, radioactivity was well distributed to most tissues, with the highest concentrations as follows: bone>bone marrow>salivary gland, thyroid, spleen, and kidney. In each of these tissues, the ratio of area under the concentration-time curve in tissue to area under the concentration-time curve in plasma was >10.

The objective of this study was to determine the tissue and corresponding serum concentration of tigecycline at selected time points in lung, colon, gallbladder tissues, bone, and synovial fluid. Samples were taken from subjects scheduled for lung, colon, gallbladder, or bone surgery, or a lumbar puncture who were given a single dose of tigecycline administered intravenously.

Pre-specified tissue/fluid sampling of either lung, colon, gallbladder, bone, and synovial fluid was performed on each subject during surgery at 4 hours, 8 hours, 12 hours, or 24 hours after the start of a single dose of 100 mg tigecycline administered over 30 minutes. Serum was collected from all subjects at hour 0 (before the first dose), approximately 30 minutes (end of infusion), and at the time corresponding to tissue/fluid collections. Tissue and serum concentration was determined according to the method set forth below.

### Investigational Parameters for Serum Samples

Samples of human serum and tissue from study subjects who had received tigecycline were analyzed according to methods that had been previously validated. Serum samples and synovial fluid (0.2 ml) were mixed with 0.6 ml internal standard in acetonitrile, the supernatant evaporated to dryness and the residue reconstituted in 200 microliters mobile phase. Aliquots (10 microliters) of the reconstituted samples were injected into an LC/MS/MS.

The data was acquired by and analyzed on PE SCIEX "Analyst" version 1.3 software. Linear regression, with 1/x² weighting was used to obtain the best fit of the data for the calibration curves. The lower limit of quantitation was 10 ng/ml for serum and synovial fluid samples, 10 ng/g for the colon and gall bladder samples, and 30 ng/g for the bone samples.

Quality control samples (2 sets) at low (25 ng/ml), medium (500 ng/ml) and high (1500 ng/ml), prepared in human serum, were analyzed with each set of serum samples. For colon, gall bladder and lung samples, two sets of quality control samples at 25, 500, and 1500 ng/g were analyzed with each set of tissue samples. For bone, two sets of quality control samples at 100, 500, and 1500 ng/g were analyzed with each set of tissue samples.

The curves were linear in the range from the 10 to 2000 ng/ml for serum and synovial fluid and from the lower limit of quantitation to 2000 ng/g for tissues. A run was considered successful if no more than two quality control samples were outside the range of 85-115% of target and no two quality control samples at the same concentration were outside that range. If two quality control samples at the same concentration were outside that range, only concentrations between the remaining quality control samples were reported.

### Materials and Methods for Serum Samples

Tigecycline was measured in human serum using an LC/MS/MS method. The primary stock solution of tigecycline was prepared at 1 mg/ml by dissolving in methanol. A secondary stock solution was prepared from the primary stock solution by diluting to a concentration of approximately 40,000 ng/ml with acetonitrile. The stock solutions were stored at -20 °C when not in use. A primary internal standard solution of tert-butyl-d9-tigecycline was prepared at a concentration of 1 mg/ml in methanol. A secondary internal standard stock solution was prepared by diluting the primary stock solution to a concentration of 100 micrograms/ml in acetonitrile with 0.1% trifluoroacetate added. The primary and secondary stock solutions were stored at -20 °C. The working internal standard was prepared by diluting to volume with acetonitrile/0.1 % trifluoroacetate. The working internal standard was stored at 4 °C when not in use. On the day of analysis, the secondary stock solution was brought to room temperature before use to prepared the standard curve working solutions. The standard curve was prepared at approximately 2000, 1600, 1000, 500, 250, 100, 50, 20, and 10 ng/ml by serial dilution in blank human serum.

The extraction procedure was as follows: to 200 microliters of calibrator, quality control or sample was added 600 microliters of internal standard working solution and vortex mixed. The samples were centrifuged for 10 minutes at 13000 rpm to separate the layers and the supernatant was transferred to a culture tube. The samples were evaporated to dryness in a Speed Vac. The residues were reconstituted by sonicating in 200 microliters of mobile phase and 10 microliters was injected in the LC/MS/MS.

The LC/MS/MS was composed of HPLC (Agilent 1100), Mass Spectrometer (Applied Biosystems API3000), Column (Aquasil C18, 50 x 2.1 mm i.s., 5micron (ThermoKeystone) with mobile phase of 16% acetonitrile, 6% methanol, 78% water, and 0.1% tetrefluoroacetate, flow rate approximately 0.35 ml/min, injection volume 10 microliters, Detector Conditions: 119 scans in period, MRM scan type, positive polarity, turbo ion spray source, at low resolution, using nitrogen at 6 psi as a nebulizer gas, a curtain gas, and a collision gas, with ion energy at 4500 mv, and ionspray temperature at 450 °C. The detector monitored tigecycline and the internal standard.

Samples were analyzed over three analytical runs. On each day of sample analysis, a complete standard curve was run, along with quality control samples and study subject samples. Samples that had a measured concentration greater than the highest calibrator were diluted by mixing 100 microliters sample with 900 microliters blank human serum and analyzing 200 microliters of the mixture as previously described. Quantification of tigecycline in serum was achieved by comparison to a standard curve prepared in the appropriate matrix and calculated using a (1/concentration)² weighting factor.

The limit of quantitation for tigecycline was 10 ng/ml. No peaks interfering with the determination of any of the tigecycline isomers were detected in any of the pre-dose samples. All calibrators and quality control samples were within range (85-115% of target). Results of samples are presented in Table 1. Results of standard curves and calibrators are presented in Table 4.

### Investigational Parameter for Tissue Samples

Stock solution and internal standard solution was prepared as per investigation parameters for serum samples above. The standard curve working solutions were prepared at approximately 10000, 8000, 5000, 2500, 500, 250, 100, and 50 mg/ml. On the day of analysis, 40 microliters of the working solutions were added to 200 mg of tissue to produce calibrators at 2000, 1600, 1000, 500, 250, 100, 50, 20, and 10 ng/ml. Canine tissue was substituted for human tissue to prepare the calibrators and quality control samples. Because of the limited availability of canine gall bladder, canine colon was used to prepare the standard curve for the analysis of human gall bladder. Colon was shown to be an appropriate substitute matrix for the analysis of gall bladder samples.

The extraction procedure was as follows: to 200 mg of calibrator, quality control or sample was added 3 ml of internal standard working solution and samples were homogenized using a hand homogenizer. The samples were centrifuged for 10 minutes at 14000 rpm to separate the layers and the supernatant was transferred to a centrifuge tube. The samples were evaporated to dryness in a Speed Vac. The residues were reconstituted by sonicating in 200 microliters of mobile phase and 10 microliters was injected into the LC/MS/MS. LC/MS/MS conditions were the same as those used to analyze serum samples. Synovial fluid samples were extracted in the same manner as serum samples.

Samples were analyzed over several analytical runs. On each day of sample analysis, a complete standard curve was run, along with quality control samples and tissues. The standard curve was prepared in the substitute matrix appropriate to the tissue samples being analyzed. Samples which had a measured concentration greater than the highest calibrator (200 ng/g) were homogenized with internal standard at 10 or 20 times the concentration use for the standard curve. An aliquot (300 microliters) (10 fold dilution) was evaporated to dryness and the samples were reconstituted so that the peak area ratios and peak areas were within the range of the standard curve.

Quantification of tigecycline in tissues was achieved by comparison to a standard curve prepared in the appropriate matrix and calculated using a (1/concentration)² weighting factor. For synovial fluid, the calibrators were prepared in phosphate-buffered saline. A second set of calibrators was prepared in an artificial synovial fluid composed of the following components: 100 mmol/L glucose, 2.03-2.26 g/L hyaluronate and approximately 8 g/L albumin adjusted to pH 7.4. The calibration curve prepared in PBS and the recovery, a correction factor was calculated by performing a linear regression of determined concentrations of artificial synovial fluid samples from the PBS curve verses the theoretical concentration of those samples using a power equation (y=y0 +ax^{b}). Because the determined concentration of study subject samples was in the low range of the calibration curve, only the calibrators from 20 to 500 ng/ml were used to calculate this regression. The results of this regression showed a strong correlation (r² = 0.9996) and back-calculated concentrations of the ASF calibrators were between 94 and 122% of their target values over the complete range of the standard curve (20-2000ng/ml). The regression equation was then applied to the concentrations of study subject samples from the PBS standard curve and the corrected concentration of tigecycline in synovial fluid samples was determined.

The limit of quantitation for tigecycline was 10 ng/ml. Measurable concentrations of tigecycline were found in all matrices analyzed. All calibrators and quality control samples at concentrations similar to the samples were within range (85-115% of target). Results of samples are presented in Table 4 (tissues) and Table 5 (synovial fluid).

### Results

The data demonstrate a rapid distribution phase, with a prolonged half-life and a high volume of distribution at steady state. They further establish the penetration of bone, synovial fluid, lung, gall bladder, and colon in human subjects. Additionally, concentrations in synovial fluid show rapid distribution and prolonged retention of tigecycline as compared to data from serum at similar times.

**TABLE 3**

| **Results of Serum Analysis** | | | | | | | | |
|---|---|---|---|---|---|---|---|---|
| | **Calculated Calculated Calculated** | | | | | | | |
| | **Sample Concentration Time** | | | **Sample Concentration Time** | | | **Sample Concentration Time** | |
| **ID.** | **{n& ml}** | **Hour** | **I.D.** | **fnpJmlZ** | **Hour** | **I.D** | **fnpJmli** | **Hour** |
| 1A | BQL' | 0 | 22A | BQL | 0 | 40A | BQL | 0 |
| 1B | 5450 | 0.5 | 22B | 1810 | 0.5 | 40B | 1950 | 0.5 |
| 1C | 81.6 | 24 | 22C | 251 | 4 | 40C | 80.6 | 24 |
| 2A | BQL | 0 | 23A | BQL | 0 | 41A | BQL | 0 |
| 2B | 1080 | 0.5 | 23B | 1530 | 0.5 | 41 B | 2580 | 0.5 |
| 2C | 151 | 4 | 23C | 74.4 | 24 | 41C | 191 | 12 |
| 4A | BQL | 0 | 24A | BQL | 0 | 42A | BQL | 0 |
| 4B | 1490 | 0.5 | 24B | 1650 | 0.5 | 42B | 789 | 0.5 |
| 4C | 175 | 4 | 24C | 85.6 | 12 | 42C | 198 | 8 |
| 5A | BQL | 0 | 25A | BQL | 0 | 43A | BQL | 0 |
| 5B | 1100 | 0.5 | 25B | 3550 | 0.5 | 43B | 1150 | 0.5 |
| 5C | 116 | 12 | 25C | 136 | 4 | 43C | 77.4 | 24 |
| 6A | BQL | 0 | 26A | BQL | 0 | 44A | BQL | 0 |
| 6B | 1170 | 0.5 | 26B | 878 | 0.5 | 44B | 954 | 0.5 |
| 6C | 113 | 12 | 26C | 120 | 12 | 44C | 144 | 4 |
| 7A | BQL | 0 | 27A | BQL | 0 | 45A | BQL | 0 |
| 7B | 1640 | 0.5 | 27B | 847 | 0.5 | 45B | 894 | 0.5 |
| 7C | 97.2 | 12 | 27C | 78.9 | 12 | 45C | 299 | 4 |
| 8A | BQL | 0 | 28A | BQL | 0 | 46A | BQL | 0 |
| 8B | 1710 | 0.5 | 28B | 922 | 0.5 | 46B | 1420 | 0.5 |
| 8C | 186 | 4 | 28C | 120 | 12 | 46C | 66.6 | 24 |
| 9A | BQL | 0 | 29A | BQL | 0 | 47A | BQL | 0 |
| 9B | 1860 | 0.5 | 29B | 5190 | 0.5 | 47B | 447 | 0.5 |
| 9C | 221 | 4 | 29C | 147 | 4 | 47C | 252 | 4 |
| 10A | BQL | 0 | 30A | BQL | 0 | 48A | BQL | 0 |
| 10B | 27400 | 0.5 | 30B | 1190 | 0.5 | 48B | 976 | 0.5 |
| 10C | 244 | 4 | 30C | 50.8 | 24 | 48C | 86.9 | 24 |
| 11A | BQL | 0 | 31A | BQL | 0 | 49A | BQL | 0 |
| 11B | 1320 | 0.5 | 31B | 2320 | 0.5 | 49B | 1200 | 0.5 |
| 11C | 47.2 | 12 | 31C | 166 | 4 | 49C | 102 | 12 |
| 12A | BQL | 0 | 32A | BQL | 0 | 50A | BQL | 0 |
| 12B | 6950 | 0.5 | 32B | 3550 | 0.5 | 50B | 1430 | 0.5 |
| 12C | 54.4 | 24 | 32C | 42.1 | 24 | 50C | 186 | 8 |
| 15A | BQL | 0 | 33A | BQL | 0 | 51A | BQL | 0 |
| 15B | 1960 | 0.5 | 33B | 620 | 0.5 | 51B | 726 | 0.5 |
| 15C | 250 | 4 | 33C | 43.7 | 24 | 51C | 44.7 | 24 |
| 16A | BQL | 0 | 34A | BQL | 0 | 52A | BQL | 0 |
| 16B | 741 | 0.5 | 34B | 4080 | 0.5 | 52B | 821 | 0.5 |
| 16C | 107 | 12 | 34C | 92.7 | 12 | 52C | 125 | 4 |
| 17A | BQL | 0 | 35A | BQL | 0 | 53A | BQL | 0 |
| 17B | 1110 | 0.5 | 35B | 2430 | 0.5 | 53B | 1060 | 0.5 |
| 17C | 51 | 24 | 35C | 53.6 | 24 | 53C | 209 | 4 |
| 18A | BQL | 0 | 36A | BQL | 0 | 54A | BQL | 0 |
| 18B | 761 | 0.5 | 36B | 2300 | 0.5 | 54B | 1850 | 0.5 |
| 18C | 133 | 8 | 36C | 65.7 | 24 | 54C | 206 | 8 |
| 19A | BQL | 0 | 37A | BQL | 0 | 55A | BQL | 0 |
| 19B | 1240 | 0.5 | 37B | 2415 | 0.5 | 55B | 628 | 0.5 |
| 19C | 162 | 4 | 37C | 95.7 | 12 | 55C | 431 | 4 |
| 20A | BQL | 0 | 38A | BQL | 0 | | | |
| 20B | 903 | 0.5 | 38B | 4600 | 0.5 | | | |
| 20C | 106 | 12 | 38C | 106 | 12 | | | |
| 21A | BQL | 0 | 39A | BQL | 0 | | | |
| 21B | 870 | 0.5 | 39B | 5130 | 0.5 | | | |
| 21C | 778 | 4 | 39C | 342 | 4 | | | |

| | | | | | | | | |
|---|---|---|---|---|---|---|---|---|
| ¹BQL = below quantitative limits | | | | | | | | |

**TABLE 4**

| **Results of Tissue Analysis Calculated** | | |
|---|---|---|
| **Sample Concentration** | | |
| **I.D.** | **(n2/2)** | **Tissue** |
| 001 | 8210 | Gall Bladder |
| 002 | 1560 | Gall Bladder |
| 004 | 41.6 | Bone |
| 005 | 20700 | Gall Bladder |
| 006 | 46.5 | Bone |
| 007 | 33.3 | Bone |
| 008 | 79.3 | Bone |
| 009 | 1890 | Lung |
| 010 | 141 | Bone |
| 011 | 7640 | Gall Bladder |
| 012 | BQL | Bone |
| 015 | 8400 | Gall Bladder |
| 016 | 824 | Gall Bladder |
| 017 | 93.3 | Bone |
| 018 | 3750 | Gall Bladder |
| 019 | 18900 | Gall Bladder |
| 020 | 269 | Bone |
| 021 | 86.6 | Colon |
| 022 | 50.0 | Bone |
| 023 | 1180 | Gall Bladder |
| 024 | BQL | Bone |
| 025 | 1550 | Gall Bladder |
| 026 | 91.2 | Colon |
| 027 | BQL | Bone |
| 028 | 598 | Colon |
| 029 | 3240 | Gall Bladder |
| 030 | BQL | Bone |
| 031 | 5960 | Gall Bladder |
| 032 | 938 | Gall Bladder |
| 033 | BQL | Bone |
| 034 | 3480 | Gall Bladder |
| 035 | 778 | Gall Bladder |
| 037 | 3850 | Gall Bladder |
| 038 | BQL | Bone |
| 039 | 198 | Colon |
| 040 | 1500 | Gall Bladder |
| 041 | 106 | Colon |
| 042 | 238 | Gall Bladder |
| 043 | 995 | Colon |
| 044 | 725 | Colon |
| 045 | 814 | Colon |
| 046 | BQL | Bone |
| 047 | 453 | Colon |
| 048 | BQL | Bone |
| 050 | 618 | Colon |
| 051 | 653 | Lung |
| 052 | 35.5 | Bone |
| 053 | BQL | Bone |
| 054 | 36.1 | Bone |
| 055 | 118 | Colon |

| | | |
|---|---|---|
| *BQL = below quantitative limits of the assay (<33.2 ng/ml) | | |

**TABLE 5**

| **Results of Synovial Fluid Analysis** | | |
|---|---|---|
| **Calculated** | | |
| **Sample Concentration Time I.D.** | | |
| | **(ng/ml)** | **(Hour)** |
| 4 | 39.9 | 4 |
| 6 | 62.8 | 12 |
| 8 | 159 | 4 |
| 10 | 130 | 4 |
| 12 | 46.4 | 24 |
| 20 | 111 | 12 |
| 22 | 181 | 4 |
| 24 | 65.0 | 12 |
| 30 | 37.8 | 24 |
| 33 | 25.9 | 24 |
| 38 | 65.7 | 12 |
| 46 | 55.4 | 24 |
| 48 | 45.0 | 24 |
| 52 | 70.6 | 4 |
| 54 | 70.9 | 8 |

### EXAMPLE 3: Tissue Distribution in Rats Treated with Tigecycline

This study was conducted to quantitate [¹⁴C]-tigecycline-derived radioactivity in tissues by whole body autoradiography using phosphor imaging, following a single 30-minute 3 mg/kg intravenous infusion of [¹⁴C]-tigecycline to male Sprague-Dawley and Long-Evans rats.

### Materials and Methods

Tigecycline was supplied by the Analytical Department, Wyeth-Ayerst Research, Montreal, Canada. [¹⁴C]-tigecycline was supplied by Amersham (Boston, MA). Radiochemical purity and specific activity of bulk [¹⁴C]-tigecycline was 98% and 93.6 microCi/mg, respectively.

Sterile water was used to make the intravenous dosing solution. The liquid scintillation cocktail used in counting the radioactivity in plasma and urine was Ultima Gold (Packard Instruments Co., Meriden, CT).

A Model 3078 Tri-Carb Sample Oxidizer equipped with an Oximate-80 Robotic Automatic Sampler (Canberra-Packard Co., Downers Grove, IL) was used for combustion of blood samples. Permafluor E liquid scintillation cocktail (Packard Instruments Co., Meridan, CT), Carbo-Sorb-E (Packard Instruments Co., Meridan CT) carbon dioxide absorber and de-ionized water were used to trap radioactive carbon dioxide generated by combustion of the sample in the oxidizer. Blood aliquots were transferred to combusto-cones and cover pads (Canberra-Packard Co., Downers Grove, IL) for combustion.

All radioactivity determinations (dose, blood and plasma) were made using a Tri-Carb Model 2700 TR liquid scintillation counter (Canberra-Packard Co., Downers Grove, IL) with an Ultima Gold or toluene standard curve. Counts per minute (CPM) were converted to disintegrations per minute (DPM) by use of external standards of known radioactivity. The quench of each standard was determined by the transformed spectral index of an external radioactive standard (TSIE). The lower limits of detection were defined as twice background.

Male Sprague-Dawley and Long-Evans rats were obtained from Charles River Breeding Laboratories, Raleigh, NC, and were quarantined for at least one week prior to the start of the study. The intravenous dosing solution (1.02 mg/ml) was prepared by dissolving 6.90 mg of unlabeled tigecycline and 5.30 mg of [¹⁴C]-tigecycline in 12 ml sterile water. The dosing solution was diluted and radioassayed directly in Ultima Gold scintillation counting cocktail (Packard Inc.). All determinations of total radioactivity were made with a Packard 2700 TR liquid scintillation spectrometer (Canberra-Packard Co.).

The rat body weights ranged from 0.206 to 0.301 kg. All rats received a single 30 minute intravenous infusion dose of [¹⁴C]-tigecycline via a jugular vein canula, (3 ml/kg, 3 mg/kg as active moiety, 40 microCi/kg) using a Harvard infusion pump 22 (Harvard Apparatus, Southnatick, MA). All pumps were calibrated prior to the administration of the compound. Rats were anesthetized with isoflurane prior to cardiac puncture exsanguination at the prescribed times after dosing. Sprague-Dawley and Long-Evans rats were sacrificed one per time point at 0.5, 8.5, 24, 72, 168 and 336 hr post-dose.

Control whole blood was collected from male Sprague-Dawley rats into tubes containing sodium heparin. Pooled blood was used to prepare the calibration standards and quality control samples. The standards were used to construct the standard curve for the quantification of radiolabeled drug distribution in tissues of whole blood cryosections. The quality controls, which were embedded in the same CMC block with each rat, were used for assessing intra- and inter-section variation in the thickness of rat whole-body cryosections.

A 200 microCi/ml stock solution of [¹⁴C] -glucose (New England Nuclear, Boston, MA) was serially diluted with whole blood from male Sprague-Dawley rats to obtain fourteen standards at the following concentrations: 832, 485, 250, 122, 48.6, 24.3, 12.0, 4.72, 2.36, 0.853, 0.638, 0.405, 0.327, and 0.221 nCi equiv./ml. The low, mid and high GCs concentrations were 12.39, 25.9 and 508 nCi equiv./ml.

Immediately following euthanasia, each rat was totally immersed in a bath of hexane and dry ice (-75 °C) until frozen. Each carcass was dried and stored at -30 °C until embedded. Each animal was embedded in a mold (15 cm x 45 cm) by adding low viscosity, 10% carbosymethylcellulose (CMC) and frozen by placing the stage in a hexane-dry ice mixture.

Frozen blocks were transferred to the Jung Cryomacrocut 3000 (Leica Instruments GmbH, Nussloch, Germany) and allowed to equilibrate overnight to the cryotome internal temperature for at least 12 hours before sectioning.

Each frozen rat was sagitally sectioned at -20°C. A sufficient number of sections were collected to ensure sampling of all tissues of interest. The sections were dehydrated overnight in a cryochamber and then rapidly transferred to a dessicator containing CaSO4 to prevent condensation of atmospheric moisture while equilibrating to room temperature. The sections were mounted on cardboard and labeled with [¹⁴C]-labeled black ink with a unique identification number. Radioactive ink was prepared with equal volumes of India Ink and [¹⁴C]-CL-284846 (100 microCi/ml). A small piece of Scotch tape was placed over the dried radioactive ink to prevent the contamination of the storage phosphor screens.

Phosphor imaging plates, BAS-SR 2025 (Fuji Photo Film Co., Japan) were exposed to bright visible light for 20 minutes using an IP eraser (Raytest, USA Inc., New Castle, DE) to remove background radiation. Sections and calibration blood standards were concurrently placed in direct contact with Ips and exposed for 7 days. All sections were stored at room temperature in a lead shielding box to minimize background levels. Phosphor images were generated using a Fujifilm BAS-5000 Bio-Imaging Analyzer and quantitated by MCID M2 Software, version 3.2 (Imaging Research Inc., St. Catherines, Ontario, Canada). The STDs and QCs were analyzed using the circular sampling tool in the MCID software program. Areas of interest in whole-body sections were manually outlined with the regional sampling tool to generate count data.

Radioactive concentrations in select tissues were determined by digital analysis of the resulting autoradiograms on the basis of a calibration curve. A calibration curve of stated concentrations (nCi/g) verses MCID response, photostimulated light/mm² (PSL/mm²-minus background converted to nCi/g) for each standard was generated by weighted (1/x²) linear regression analysis. The linear regression curve was then used to determine the concentration of unknown radioactivity of study samples. The regions of interest (ROI) which visually exhibited levels of radioactivity were individually outlined or autoscanned with sampling tools to obtain radioactivity concentrations. To determine the limit of quanititation for QWBAR, coefficients of variation from blood standards tested were determined, and the limit of quantitation was defined as the lowest concentration at which the coefficients of variation did not exceed 15%.

Plasma aliquots were combined with 10 ml of Ultima Gold™ scintillation counting cocktail (Packard Inc.) and directly counted. Blood samples were combusted using a Model 307 sample oxidizer (Packard Instrument Company). The resulting [¹⁴C]O₂ was trapped in Carbo-Sorb®, scintillation cocktail (PermaFlour®E+) was added, and the samples were quantitated by LSC.

Samples were counted in a Packard 2700 TR liquid scintillation spectrophotometer (Canberra-Packard Co.) for 10 minutes or 0.2 sigma. Counts per minute were converted to disintegrations per minute by use of a quench curve generated from external standards of known radioactivity. The quench of each standard and sample were determined by full spectral shift. Limit of quantitation (LOQ) for LSC was defined as two times background.

The pharmacokinetic parameters for [¹⁴C]-GAR-936-derived radioactivity were calculated using the intravenous infusion (Model 202), Non-Compartment Analysis Module of WinNonlin, ver. 1.1, (Scientific Consultants, Inc. Research Triangle Park, NC), which applies a model-independent approach and standard procedures as described in Gibaldi and Perrier. Gibaldi, *Pharmacokinetics,* 1982. In determining the mean concentration, zero was substituted for any values that were below the limit of quantitation (5.10 ng equiv./g). For IV infusion dosing, C30 min was the concentration at 30 minutes, the first sampling time point. The maximum plasma concentration (Cₘₐₓ) and the corresponding time of peak concentration following IV administration were obtained directly by numerical inspection from the individual concentration-time data. The terminal half-life was calculated by the ratio of 1n2/λz where λz is derived from the terminal slope of the concentration time curve. The area under the plasma concentration versus time curve from zero to infinity was calculated using the trapezoidal rule, where Cₗₐₛₜ is the last measurable plasma concentration. Tissue to plasma concentration ratios were calculated according to the following equation: Cₜᵢₛₛᵤₑ/Cₚₗₐₛₘₐ, where Cₜᵢₛₛᵤₑ equals the drug concentration in tissue (ng equiv./g), and Cₚₗₐₛₘₐ equals the drug concentration in plasma (ng equiv./g).

The specific activity of [¹⁴C]-tigecycline (base) was determined by gravimetric assay to be 43.94 µCi/mg (Table 1). The concentration of the dosing solution was 1.02 mg/ml. Animals received an average dose of 3.09 ± 0.11/kg compared to a target dose of 3 mg/kg.

### Results

Individual concentrations (ng equiv./g) of total radioactivity in tissues of Sprague-Dawley rats following a 3 mg/kg IV infusion of [¹⁴C]-tigecycline are represented in Table 6. Pharmacokinetic parameters in tissues are presented in Table 7. Tissue to plasma ratios are presented in Table 8.

Individual peak concentrations (Cₘₐₓ) of total radioactivity occurred at the end of infusion for virtually all of the tissues. Tissues with the highest concentrations of radioactivity were kidney (7601 ng equiv./g), liver (7300 ng equiv./g) and spleen (6627 ng equiv./g) (Table 7). The tissues with the lowest peak concentration of radioactivity were the brain (54 ng equiv./g) and eyes (108 ng equiv./g) (Table 7). Cₘₐₓ was greater than 2000 ng equiv./g for most (70%) tissues. Tigecycline-derived radioactivity at Cₘₐₓ was lower in plasma than in all tissues, except brain, eyes, fat and testes (Table 7). By 24 hrs, all tissues had higher concentrations of [¹⁴C]-tigecycline-derived radioactivity (Table 6) than plasma except eyes.

Individual tissues concentrations of radioactivity at 168 hours for most tissues declined to 1% or less, relative to their Cₘₐₓ, with the exception of bone, kidney, liver, skin, spleen and thyroid (Table 6). By 336 hours, most tissues had concentrations below the quantitation limit (5.10 ng. equiv./g) except bone, kidney, skin and thyroid. However, the concentrations in these tissues (bone, thyroid, kidney and skin) were greatly reduced from Cₘₐₓ In general, tissue concentrations of [¹⁴C]-tigecycline-derived radioactivity in bone, kidney, skin and thyroid at 336 hours were 19%, 0.18%, 0/43% and 6% of Cₘₐₓ, respectively.

Using AUC as a measure of tissue burden, the bone and thyroid had a much greater burden than any other tissues. The highest AUC values were in the bone (794704 ng eq·hr/g), thyroid (330047 ng eq·hr/g), salivary gland (110979 ng eq·hr/g), kidney (70704 ng eq·hr/g), thyroid (33047 ng eq·hr/g), spleen (70522 ng eq·hr/g) and liver (53527 ng eq·hr/g). The tissues with lowest burden were the brain (2865 ng eq·hr/g), fat (3500 ng eq·hr/g) and testes (10303 ng eq·hr/g). AUC exposure in bone was two-times higher than the next highest tissue (thyroid). Tissue:plasma AUC ratio values were greater than one for the majority of the tissues (Table 7).

The terminal half-life for [¹⁴C]-tigecycline-derived radioactivity ranged from a low of 5 hours in the fat to more than 200 hours in the bone and thyroid, compared with a plasma t_{1/2} of 24 hours (Table 7). Tissues with the longest elimination half-life were thyroid (804 hours), bone (217 hours), skin (182 hours) and kidney (118 hours) (Table 7).

The tissue:plasma concentration ratios (Table 8) were greater than one for the majority of tissues, with the exception of brain, eyes, testes, and fat at the 0.5 and 8.5 hour time points. At 24 hours, all ratios were greater than one. The highest tissue to plasma ratios occurred for some tissues at 72 hours:bone (414), thyroid (56), skin (19.3), spleen (16.7), and kidney (11.1). The blood:plasma ratios were greater than one for all time points, suggesting that there was substantial partitioning of [¹⁴C]-tigecycline-derived radioactivity into blood cells.

The distribution of [¹⁴C]-tigecycline-derived radioactivity to melanin-containing tissues (skin and uveal tract) in Long-Evans rats was also evaluated up to 336 hours post-dose. Blood and plasma concentrations of [¹⁴C]-tigecycline-derived radioactivity in Long-Evans rats were similar to Sprague-Dawley rats (Tables 2 and 5). Peak radioactivity concentrations (Cₘₐₓ) were observed at the end of infusion (0.5 hour) for skin, uveal tract, plasma and blood (Table 9). The Cₘₐₓ of [¹⁴C]-tigecycline-derived radioactivity in skin and uveal tract was 1997 and 2502 ng equiv./g, respectively. The AUC of [¹⁴C] [¹⁴C]-tigecycline-derived radioactivity in skin and uveal tract were 109296 and 233288 ng equiv·hr/g, respectively. The terminal half-lives for skin and uveal tract were 473 and 20 hours, respectively (Table 10). The half-life values are of questionable meaning since the elimination phases in the concentration-time profile could not be identified with certainty. This is also reflected in the extrapolation of AUC data for uveal tract and skin.

The tissue:plasma concentration ratios were greater than one for skin and uveal tract at all time points (Table 7). The overall highest tissue to plasma ratios occurred at 72 hours in skin (179) and uveal tract (393). The tissue:plasma AUC ratios were 8.45 and 18.0 for skin and uveal tract, respectively, and indicate that these tissues selectively retain significant concentrations of [¹⁴C]-tigecycline-derived radioactivity. The data suggest that radioactivity selectively partitioned in the melanin-containing region of the rat eye. Mean tissue concentrations of radioactivity at 336 hours for skin and uveal tract declined to 8 and 1% of Cₘₐₓ, respectively.

**Table 6**

| **Mean Concentrations of Total Radioactivity in Tissues Following a Single 30 Minute Infusion of [¹⁴C]-Tigecycline in Male Sprague-Dawley Rats** | | | | | | |
|---|---|---|---|---|---|---|
| Tissue Type | 0.5 Hrs | 8.5 Hrs. | 24 Hrs. | 72 Hrs. | 168 Hrs. | 336 Hrs. |
| Blood | 1277 | 624 | 44.5 | 11.8 | 1.87 | <1.03 |
| Plasma | 895 | 504 | 14.8 | 4.31 | <1.03 | <1.03 |
| Adrenal Gland | 3580 | 941 | 68.9 | 19.3 | <5.10 | <5.10 |
| Bone | 3312 | 3794 | 2711 | 1787 | 1526 | 720 |
| Bone Marrow | 4376 | 1562 | 291 | 22.5 | <5.10 | <5.10 |
| Brain | 35.8 | 54.3 | 35.8 | 13.3 | <5.10 | <5.10 |
| Eyes | 106 | 108 | 36.6 | 6.76 | <5.10 | <5.10 |
| Fat | 450 | 144 | <5.1 | <5.10 | <5.10 | <5.10 |
| Heart | 5657 | 1138 | 69.9 | 6.98 | <5.10 | <5.10 |
| Kidney | 7601 | 1725 | 140 | 47.9 | 41.3 | 13.9 |
| Liver | 7300 | 1192 | 160 | 22.7 | 13.3 | <5.10 |
| Lung | 2981 | 496 | 73.1 | 13.5 | <5.10 | <5.10 |
| Lymph Node | 3473 | 1276 | 180 | 29.1 | <5.10 | <5.10 |
| Muscle | 2260 | 1863 | 85.8 | 6.24 | <5.10 | <5.10 |
| Pancreas | 4437 | 971 | 70.0 | 7.41 | <5.10 | <5.10 |
| Pituitary | 3693 | 2014 | 144 | 18.8 | <5.10 | <5.10 |
| Salivary Gland | 5771 | 6313 | 300 | 31.6 | <5.10 | <5.10 |
| Skin | 1929 | 577 | 249 | 83.1 | 7.32 | 8.34 |
| Spleen | 6627 | 1691 | 476 | 72.0 | 18.8 | <5.10 |
| Testes | 347 | 361 | 119 | 16.4 | <5.10 | <5.10 |
| Thymus | 2528 | 1590 | 158 | 15.3 | <5.10 | <5.10 |
| Thyroid | 2992 | 1762 | 354 | 242 | 218 | 187 |

**Table 7**

| **Pharmacokinetic Parameters of Total Radioactivity in Tissues Following a Single 30 Minute Infusion of [¹⁴C]-Tigecycline in Male Sprague-Dawley Rats** | | | | | |
|---|---|---|---|---|---|
| Tissue Type | Cmax Ng equiv/g | T_{1/2} | AUC Ng eq hr/g | AUC Ng eq hr/g | Tissue:Plasma AUC |
| Blood | 1277 | 105 | 12063 | 1261 | 1.15 |
| Plasma | 895 | 24 | 10643 | 10652 | 1.00 |
| Adrenal Gland | 3580 | 13 | 29153 | 29515 | 2.77 |
| Bone | 3794 | 217 | 569498 | 794704 | 74.6 |
| Bone Marrow | 4376 | 11 | 47116 | 47468 | 4.46 |
| Brain | 54 | 32 | 2256 | 2865 | 0.269 |
| Eyes | 108 | 17 | 3060 | 3221 | 0.302 |
| Fat | 450 | 5 | 2489 | 3500 | 0.329 |
| Heart | 5657 | 9 | 40083 | 40179 | 3.77 |
| Kidney | 7601 | 118 | 68333 | 70704 | 6.64 |
| Liver | 7300 | 44 | 52676 | 53527 | 5.03 |
| Lung | 2981 1 | 13 | 21263 | 21523 | 2.02 |
| Lymph Node | 3473 | 13 | 36478 | 37010 | 3.47 |
| Muscle | 2260 | 8 | 34833 | 34910 | 3.28 |
| Pancreas | 4437 | 10 | 32908 | 33014 | 3.10 |
| Pituitary | 3693 | 10 | 44882 | 45164 | 4.24 |
| Salivary Gland | 6313 | 9 | 110558 | 110979 | 10.4 |
| Skin | 1929 | 182 | 31819 | 36471 | 3.42 |
| Spleen | 6627 | 33 | 69638 | 70522 | 6.62 |
| Testes | 361 | 15 | 9975 | 10323 | 0.97 |
| Thymus | 2528 | 10 | 35204 | 35430 | 3.33 |
| Thyroid | 2992 | 804 | 113022 | 330047 | 31.0 |

**Table 8**

| **Tissue:Plasma Ratio Following a Single 30 Minute Infusion of [¹⁴C]-Tigecycline in Male Sprague-Dawley Rats** | | | | | | |
|---|---|---|---|---|---|---|
| Tissue Type | 0.5 Hrs | 8.5 Hrs. | 24 Hrs. | 72 Hrs. | 168 Hrs. | 336 Hrs. |
| Blood | 1.43 | 1.24 | 3.01 | 2.74 | NA | NA |
| Plasma | 1.00 | 1.00 | 1.00 | 1.00 | NA | NA |
| Adrenal Gland | 4.00 | 1.87 | 4.67 | 4.47 | NA | NA |
| Bone | 3.70 | 7.53 | 184 | 414 | NA | NA |
| Bone Marrow | 4.89 | 3.10 | 19.7 | 5.21 | NA | NA |
| Brain | 0.040 | 0.108 | 2.42 | 3.10 | NA | NA |
| Eyes | 0.118 | 0.215 | 2.46 | 1.57 | NA | NA |
| Fat | 0.50 | 0.287 | NA | NA | NA | NA |
| Heart | 6.32 | 2.26 | 4.74 | 1.62 | NA | NA |
| Kidney | 8.49 | 3.42 | 9.49 | 11.1 | NA | NA |
| Liver | 8.16 | 2.37 | 10.8 | 5.26 | NA | NA |
| Lung | 3.33 | 0.984 | 4.96 | 3.13 | NA | NA |
| Lymph Node | 3.88 | 2.53 | 12.2 | 6.75 | NA | NA |
| Muscle | 2.52 | 3.70 | 5.801 | 1.45 | NA | NA |
| Pancreas | 4.96 | 1.93 | 4.75 | 1.72 | NA | NA |
| Pituitary | 4.13 | 4.00 | 9.79 | 4.36 | NA | NA |
| Salivary Gland | 6.45 | 12.53 | 20.3 | 7.32 | NA | NA |
| Skin | 2.15 | 1.14 | 16.9 | 19.3 | NA | NA |
| Spleen | 7.40 | 3.36 | 32.3 | 16.7 | NA | NA |
| Testes | 0.388 | 0.716 | 8.08 | 3.80 | NA | NA |
| Thymus | 2.82 | 3.16 | 10.7 | 3.55 | NA | NA |
| Thyroid | 3.34 | 3.50 | 24.0 | 56.0 | NA | NA |

**Table 9**

| **Mean Concentration (ng equiv./g) of Total Radioactivity in Tissues Following a Single 30 Minute Infusion of [¹⁴C]-Tigecycline in Male Long-Evans Rats** | | | | | |
|---|---|---|---|---|---|
| Tissue Type | 0.5 Hrs | 24 Hrs. | 72 Hrs. | 168 Hrs. | 336 Hrs. |
| Blood | 1296 | 340 | 11.8 | 2.72 | 1.24 |
| Plasma | 975 | 70.5 | 4.31 | <1.03 | <1.03 |
| Uveal Tract | 1997 | 124 | 96.5 | 74.8 | 19.3 |
| Skin | 2502 | 2363 | 1787 | 351 | 211 |

**Table 10**

| **Pharmacokinetic Parameters of Total Radioactivity in Tissues Following a Single 30 Minute Infusion of [¹⁴C]-Tigecycline in Male Long-Evans Rats** | | | | | |
|---|---|---|---|---|---|
| Tissue Type | Cmax Ng equiv/g | T_{1/2} | AUC Ng eq hr/g | AUC Ng eq hr/g | Tissue:Plasma AUC |
| Blood | 1296 | 62 | 21843 | 21954 | 1.70 |
| Plasma | 975 | 19 | 12923 | 12938 | 1.00 |
| Skin | 1997 | 473 | 58287 | 109296 | 8.45 |
| Uveal Tract | 2502 | 201 | 131346 | 233288 | 18.0 |

**Table 11**

| **Tissue:Plasma Ratio Following a Single 30 Minute Infusion of [¹⁴C]-Tigecycline in Male Long-Evans Rats** | | | | | |
|---|---|---|---|---|---|
| Tissue Type | 0.5 Hrs | 24 Hrs. | 72 Hrs. | 168 Hrs. | 336 Hrs. |
| Blood | 1.33 | 4.81 | 5.05 | 5.05 | NA |
| Plasma | 1.00 | 1.00 | 1.00 | 1.00 | NA |
| Skin | 2.05 | 8.76 | 179 | 179 | NA |
| Uveal Tract | 2.57 | 25.7 | 393 | 393 | NA |

### Discussion

The distribution of radioactivity to tissues was evaluated following a single thirty minute intravenous infusion (3 mg/kg) of [¹⁴C]-tigecycline to Sprague-Dawley and Long-Evans rats. Radioactivity was distributed to tissues rapidly, with Cmax, observed at the end of infusion (0.5 hr) for most tissues. Tissue concentrations were similar to a study conducted previously by the tissue dissection method. The extensive distribution of tigecycline into a variety of tissues is suggestive of a very large volume of distribution. This finding confirms the previous observation of a high volume of distribution in rats and dogs. In general, the elimination of radioactivity from most of the tissues was slower than the rate from plasma.

The concentrations of [¹⁴C]-tigecycline-derived radioactivity in tissues of Sprague-Dawley rats was higher than plasma at most of the time points. Tissue concentrations of radioactivity at 168 hours for most tissues decline to 1% or less, relative to their end of infusion values. By 336 hours, concentrations in bone, thyroid, kidney and skin declined to 19%, 6.25%, 0.18% and 0.43% of Cmax values, respectively.

Tissues with the highest levels of exposure in Sprague-Dawley rats, as indicated by the mean AUC values, were bone, thyroid, salivary glands, kidney and spleen. The elimination half-lives were quite long (5 to 217 hours), with bone, skin and thyroid having the longest elimination half-lives. The value of half-life for the thyroid tissue is questionable since elimination phases in the concentration-time profile could not be identified with certainty. This is also reflected in the extrapolation of AUC data to AUC.

The tissue to plasma and blood to plasma ratios were greater than one for all time points, suggesting that there was substantial partitioning of [¹⁴C]-tigecycline-derived radioactivity into tissues and blood cells. The tissue to plasma ratio results from this study are similar to tissue to plasma ratio results from the rat following IV dose of minocycline.

While not being bound by theory, the high radioactivity concentrations in the bone may be due to chelation of tigecycline to calcium. The ability of tetracyclines (minocycline, choloretetracyclines) to form chelation complexes with calcium or other metal ions and thereby adhere to bone has been described in the literature. In the current study [¹⁴C]-tigecycline-derived radioactivity was significantly retained in bone, with an AUC of 794704 ng equiv·hr/g. This value is approximately 75-fold greater than plasma. An apparent elimination half-life of 217 hours was also observed in bone. The retention of radioactivity in bone may account for the somewhat incomplete recovery (89.4 ± 2.50%) of [¹⁴C]-tigecycline in a mass balance study in male Sprague-Dawley rats observed following a 5 mg/kg intravenous dose. Exposure (AUC) in bone was 2.5-fold higher than the next highest tissue (thyroid). [¹⁴C]-tigecycline-derived radioactivity also showed a strong affinity and long half-life for bone and thyroid tissues which is also similar to other known tetracyclines.

[¹⁴C]-tigecycline-derived radioactivity concentrations were detectable up to 336 hours in the kidney and were higher than those of the other tissues except for the bone and thyroid. However, in the mass balance study as well as biliary and urinary excretion study, most of the [¹⁴C]-tigecycline-derived radioactivity was excreted in the first 48 hours, suggesting that some [¹⁴C]-tigecycline-derived radioactivity may be binding with high affinity to the kidney tissue. Binding to kidney tissue is also known with tetracyclines.

As determined by QWBAR, radioactivity present in rat ocular tissues was selectively partitioned only into the melanin-containing tissues of the uveal tract in addition to the skin in the Long-Evans rats. The uveal tract had relatively high concentrations of radioactivity at all time points after 0.5 hours, suggesting a significant level of exposure and a long half-life. In a previously conducted study using the tissue dissection method, evaluation of intact eyeball revealed radioactivity was present in this organ; however, it was not possible to associate the location of this radioactivity to any specific ocular tissues.

Concentrations of the 14 standards and 28 QCS determined by conventional liquid scintillation counting (LSC) was similar to that of QWBAR evaluations for these same standards. Exposure of these standards to 14 different storage phosphor screens resulted in a reliable MCID response that correlated with the LSC determined specific activities, suggesting that intra-day and inter-day variability was very low. The CV and accuracy of the QWBAR method were within acceptable limits (≤ 20%). The reproducibility of the MCID response and good correlation of the specific activities between conventional LSC and QWBAR demonstrated that the RBC standards were of uniform concentration of radioactivity. The variability observed in this study was considered to be related to various aspects of cryosectioning, QWBAR technique and imaging analysis. QWBAR was shown to be reproducible with a sensitivity of 0.221 nCi/g (lower limit of quantitation). The dynamic range was linear across four orders of magnitude from 0.221 to 832 nCi/g.

In conclusion, tissue concentrations of [¹⁴C]-tigecycline-derived radioactivity were higher for most tissues compared to plasma concentrations. In general, the elimination of radioactivity from most of tissues was slower than the rate from plasma. AUC was higher for most tissues that plasma, suggesting that most of the tissues were slow in eliminating [¹⁴C]-tigecycline-derived radioactivity.

## Claims

1. Use of a pharmacologically effective amount of tigecycline for the manufacture of a medicament for the treatment of an infection in bone or bone marrow in a mammal.

2. The use of claim 1, wherein the treatment further comprises administering an antimicrobial agent selected from the group consisting of rifamycin, rifampin, rifapentine, rifaximin, or streptovaricin.

3. The use of claim 2 where the antimicrobial is rifampin.

4. The use of claim 1, 2 or 3 where the infection is comprised of a pathogen selected from the group consisting of gram negative bacteria, gram positive bacteria, anaerobic bacteria, and aerobic bacteria.

5. The use of claim 4 where the pathogen is selected from the group consisting of *Staphylococcus, Acinetobacter, Mycobacterium, Haemophilus, Salmonella, Streptococcus, Enterobacteriaceae, Enterococcus, Escherichia, Pseudomonas, Neisseria, Rickettsia, Pneumococci, Prevotella, Peptostreptococci, Bacteroides Legionella, beta-haemolytic streptococci,* group B streptococcus and *spirochaetes.*

6. The use of claim 5 wherein the infection is comprised of *Neisseria, Mycobacterium, Staphylococcus,* and *Haemophilus.*

7. The use of claim 6 wherein the infection is comprised of *Neisseria meningitidis, Mycobacterium tuberculosis, Mycobacterium leprae, Staphylococcus aureus, Staphylococcus epidermidis,* or *Haemophilus influenzae.*

8. The use of claim 4 where the pathogen exhibits antibiotic resistance.

9. The use of claim 8 where the antibiotic resistance is selected from the group consisting of methicillin resistance, glycopeptide resistance, tetracycline resistance, oxytetracycline resistance, doxycycline resistance; chlortetracycline resistance, minocycline resistance, glycylcycline resistance, cephalosporin resistance, ciprofloxacin resistance, nitrofurantoin resistance, trimethoprim-sulfa resistance, piperacillin/tazobactam resistance, moxifloxacin resistance, vancomycin resistance, teicoplanin resistance, penicillin resistance, and macrolide resistance.

10. The use of claim 9 where the glycopeptide resistance is vancomycin resistance.

11. The use of claim 5 where the pathogen is selected from the group consisting of *Staphylococcus aureus, Staphylococcus epidermidis, Streptococcus pneumoniae, or Streptococcus pyogenes.*

12. The use of claim 11 where the infection is comprised of *Staphylococcus aureus.*

13. The use of claim 12 where the *Staphylococcus aureus* exhibits an antibiotic resistance selected from the group consisting of glycopeptide resistance, tetracycline resistance, minocycline resistance, methicilin resistance, vancomycin resistance and resistance to a glycylcycline antibiotic other than tigecycline.

14. The use of claim 5 where the infection is comprised of *Acinetobacter baumannii.*

15. The use of claim 14 where the *Acinetobacter baumanii* exhibits an antibiotic resistance selected from the group consisting of cephalosporin resistance, ciprofloxacin resistance, nitrofurantoin resistance, trimethoprim-sulfa resistance, and piperacillin/tazobactam resistance.

16. The use of claim 5 where the infection is comprised of *Mycobacterium abscessus.*

17. The use of claim 16 where the *Mycobacterium abscessus* exhibits moxifloxacin resistance.

18. The use of claim 5 where the infection is comprised of *Haemophilus influenzae.*

19. The use of claim 5 where the infection is comprised of *Enterococcus faecium.*

20. The use of claim 5 where the infection is comprised of *Escherichia coli.*

21. The use of claim 5 where the infection is comprised of *Neisseria gonorrhoeae.*

22. The use of claim 5 where the infection is comprised of *Rickettsia prowazekii, Rickettsia typhi,* or *Rickettsia rickettsii.*

23. The use of claim 4 wherein the infection causes osteomyelitis.

24. Use of a pharmacologically effective amount of tigecycline for the manufacture of a medicament for the treatment of a joint infection or an infection of surrounding tissues of the joint in a mammal.

25. The use of claim 24, wherein the treatment further comprises administering an antimicrobial agent selected from the group consisting of rifamycin, rifampin, rifapentine, rifaximin, or streptovaricin.

26. The use of claim 25 where the antimicrobial is rifampin.

27. The use of claim 24, 25 or 26 where the infection is comprised of a pathogen selected from the group consisting of gram negative bacteria, gram positive bacteria, anaerobic bacteria, and aerobic bacteria.

28. The use of claim 27 where the pathogen is selected from the group consisting of *Staphylococcus, Acinetobacter, Mycobacterium, Haemophilus, Salmonella, Streptococcus, Enterobacteriaceae, Enterococcus, Escherichia, Pseudomonas, Neisseria, Rickettsia, Pneumococci, Prevotella, Peptostreptococci, Bacteroides Legionella, beta-haemolytic streptococci,* group B streptococcus and *spirochaetes.*

29. The use of claim 28 wherein the infection is comprised of *Neisseria, Mycobacterium, Staphylococcus,* and *Haemophilus.*

30. The use of claim 29 wherein the infection is comprised of *Neisseria meningitidis, Mycobacterium tuberculosis, Mycobacterium leprae, Staphylococcus aureus, Staphylococcus epidermidis,* or *Haemophilus influenzae.*

31. The use of claim 27 where the pathogen exhibits antibiotic resistance.

32. The use of claim 31 where the antibiotic resistance is selected from the group consisting of methicillin resistance, glycopeptide resistance, tetracycline resistance, oxytetracycline resistance, doxycycline resistance; chlortetracycline resistance, minocycline resistance, glycylcycline resistance, cephalosporin resistance, ciprofloxacin resistance, nitrofurantoin resistance, trimethoprim-sulfa resistance, piperacillin/tazobactam resistance, moxifloxacin resistance, vancomycin resistance, teicoplanin resistance, penicillin resistance, and macrolide resistance.

33. The use of claim 32 where the glycopeptide resistance is vancomycin resistance.

34. The use of claim 28 where the pathogen is selected from the group consisting of *Staphylococcus aureus, Staphylococcus epidermidis, Streptococcus pneumoniae,* or *Streptococcus pyogenes.*

35. The use of claim 34 where the infection is comprised of *Staphylococcus aureus.*

36. The use of claim 35 where the *Staphylococcus aureus* exhibits an antibiotic resistance selected from the group consisting of glycopeptide resistance, tetracycline resistance, minocycline resistance, methicilin resistance, vancomycin resistance and resistance to a glycylcycline antibiotic other than tigecycline.

37. The use of claim 28 where the infection is comprised of *Acinetobacter baumannii.*

38. The use of claim 37 where the *Acinetobacter baumanii* exhibits an antibiotic resistance selected from the group consisting of cephalosporin resistance, ciprofloxacin resistance, nitrofurantoin resistance, trimethoprim-sulfa resistance, and piperacillin/tazobactam resistance.

39. The use of claim 28 where the infection is comprised of *Mycobacterium abscessus.*

40. The use of claim 39 where the *Mycobacterium abscessus* exhibits moxifloxacin resistance.

41. The use of claim 28 where the infection is comprised of a pathogen selected from the group consisting of *Haemophilus influenzae, Enterococcus faecium, Escherichia coli, Neisseria gonorrhoeae, Rickettsia prowazekii, Rickettsia typhi,* or *Rickettsia rickettsii.*

42. The use of claim 27 wherein the joint infection or infection of the surrounding tissues of the joint cause septic arthritis.

43. The use of claim 1 or 2, wherein the bone or bone marrow infection cause osteomyelitis.

44. The use of claim 24 or 25, wherein the joint infection or infection of the tissues surrounding the joint cause septic arthritis.

## Patentansprüche

1. Verwendung einer pharmakologisch wirksamen Menge an Tigecyclin für die Herstellung eines Arzneimittels zur Behandlung einer Infektion im Knochen oder Knochenmark in einem Säuger.

2. Verwendung gemäß Anspruch 1, wobei die Behandlung weiter die Verabreichung einer antimikrobiellen Substanz ausgewählt aus der Gruppe bestehend aus Rifamycin, Rifampin, Rifapentin, Rifaximin oder Streptovaricin umfasst.

3. Verwendung gemäß Anspruch 2, wobei die antimikrobielle Substanz Rifampin ist.

4. Verwendung gemäß Anspruch 1, 2 oder 3, wobei die Infektion einen Krankheitserreger umfasst, der aus der Gruppe bestehend aus Gram-negativen Bakterien, Gram-positiven Bakterien, anaeroben Bakterien und aeroben Bakterien ausgewählt ist.

5. Verwendung gemäß Anspruch 4, wobei der Krankheitserreger ausgewählt ist aus der Gruppe bestehend aus *Staphylococcus, Acinetobacter, Mycobacterium, Haemophilus, Salmonella, Streptococcus,* Enterobacteriaceae, *Enterococcus, Escherichia, Pseudomonas, Neisseria, Rickettsia, Pneumococci, Prevotella, Peptostreptococci, Bacteroides, Legionella,* beta-hämolysierenden *Streptococci, Streptococcus* der Gruppe B und Spirochaeten.

6. Verwendung gemäß Anspruch 5, wobei die Infektion *Neisseria, Mycobacterium, Staphylococcus* und *Haemophilus* umfasst.

7. Verwendung gemäß Anspruch 6, wobei die Infektion *Neisseria meningitidis, Mycobacterium tuberculosis, Mycobacterium leprae, Staphylococcus aureus, Staphylococcus epidermidis* oder *Haemophilus influenzae* umfasst.

8. Verwendung gemäß Anspruch 4, wobei der Krankheitserreger Antibiotikum-Resistenz besitzt.

9. Verwendung gemäß Anspruch 8, wobei die Antibiotikum-Resistenz ausgewählt ist aus der Gruppe bestehend aus Methicillin-Resistenz, Glykopeptid-Resistenz, Tetracyclin-Resistenz, Oxytetracyclin-Resistenz, Doxycyclin-Resistenz, Chlortetracyclin-Resistenz, Minocyclin-Resistenz, Glycylcyclin-Resistenz, Cephalosporin-Resistenz, Ciprofloxacin-Resistenz, Nitrofurantoin-Resistenz, Trimethoprim/Sulfa-Resistenz, Piperacillin/Tazobactam-Resistenz, Moxifloxacin-Resistenz, Vancomycin-Resistenz, Teicoplanin-Resistenz, Penicillin-Resistenz und Makrolid-Resistenz.

10. Verwendung gemäß Anspruch 9, wobei die Glykopeptid-Resistenz eine Vancomycin-Resistenz ist.

11. Verwendung gemäß Anspruch 5, wobei der Krankheitserreger ausgewählt ist aus der Gruppe bestehend aus *Staphylococcus aureus, Staphylococcus epidermidis, Streptococcus pneumoniae* oder *Streptococcus pyogenes.*

12. Verwendung gemäß Anspruch 11, wobei die Infektion *Staphylococcus aureus* umfasst.

13. Verwendung gemäß Anspruch 12, wobei der *Staphylococcus aureus* eine Antibiotikum-Resistenz besitzt, die ausgewählt ist aus der Gruppe bestehend aus Glykopeptid-Resistenz, Tetracyclin-Resistenz, Minocyclin-Resistenz, Methicillin-Resistenz, Vancomycin-Resistenz und Resistenz gegenüber einem Glycylcyclin-Antibiotikum, das nicht Tigecyclin ist.

14. Verwendung gemäß Anspruch 5, wobei die Infektion *Acinetobacter baumannii* umfasst.

15. Verwendung gemäß Anspruch 14, wobei der *Acinetobacter baumannii* eine Antibiotikum-Resistenz besitzt, die ausgewählt ist aus der Gruppe bestehend aus Cephalosporin-Resistenz, Ciprofloxacin-Resistenz, Nitrofurantoin-Resistenz, Trimethoprim/Sulfa-Resistenz und Piperacillin/Tazobactam-Resistenz.

16. Verwendung gemäß Anspruch 5, wobei die Infektion *Mycobacterium abscessus* umfasst.

17. Verwendung gemäß Anspruch 16, wobei *Mycobacterium abscessus* Moxifloxacinresistent ist.

18. Verwendung gemäß Anspruch 5, wobei die Infektion *Haemophilus influenzae* umfasst.

19. Verwendung gemäß Anspruch 5, wobei die Infektion *Enterococcus faecium* umfasst.

20. Verwendung gemäß Anspruch 5, wobei die Infektion *Escherichia coli* umfasst.

21. Verwendung gemäß Anspruch 5, wobei die Infektion *Neisseria gonorrhoeae* umfasst.

22. Verwendung gemäß Anspruch 5, wobei die Infektion *Rickettsia prowazekii, Rickettsia typhi* oder *Rickettsia rickettsii* umfasst.

23. Verwendung gemäß Anspruch 4, wobei die Infektion eine Osteomyelitis verursacht.

24. Verwendung einer pharmakologisch wirksamen Menge an Tigecyclin für die Herstellung eines Arzneimittels zur Behandlung einer Gelenkinfektion oder einer Infektion der das Gelenk umgebenden Gewebe in einem Säuger.

25. Verwendung gemäß Anspruch 24, wobei die Behandlung weiter die Verabreichung einer antimikrobiellen Substanz ausgewählt aus der Gruppe bestehend aus Rifamycin, Rifampin, Rifapentin, Rifaximin oder Streptovaricin umfasst.

26. Verwendung gemäß Anspruch 25, wobei die antimikrobielle Substanz Rifampin ist.

27. Verwendung gemäß Anspruch 24, 25 oder 26, wobei die Infektion einen Krankheitserreger umfasst, der aus der Gruppe bestehend aus Gram-negativen Bakterien, Gram-positiven Bakterien, anaeroben Bakterien und aeroben Bakterien ausgewählt ist.

28. Verwendung gemäß Anspruch 27, wobei der Krankheitserreger ausgewählt ist aus der Gruppe bestehend aus *Staphylococcus, Acinetobacter, Mycobacterium, Haemophilus, Salmonella, Streptococcus,* Enterobacteriaceae, *Enterococcus, Escherichia, Pseudomonas, Neisseria, Rickettsia, Pneumococci, Prevotella, Peptostreptococci, Bacteroides, Legionella,* beta-hämolysierenden *Streptococci, Streptococcus* der Gruppe B und Spirochaeten.

29. Verwendung gemäß Anspruch 28, wobei die Infektion *Neisseria, Mycobacterium, Staphylococcus* und *Haemophilus* umfasst.

30. Verwendung gemäß Anspruch 29, wobei die Infektion *Neisseria meningitidis, Mycobacterium tuberculosis, Mycobacterium leprae, Staphylococcus aureus, Staphylococcus epidermidis* oder *Haemophilus influenzae* umfasst.

31. Verwendung gemäß Anspruch 27, wobei der Krankheitserreger Antibiotikum-Resistenz besitzt.

32. Verwendung gemäß Anspruch 31, wobei die Antibiotikum-Resistenz ausgewählt ist aus der Gruppe bestehend aus Methicillin-Resistenz, Glykopeptid-Resistenz, Tetracyclin-Resistenz, Oxytetracyclin-Resistenz, Doxycyclin-Resistenz, Chlortetracyclin-Resistenz, Minocyclin-Resistenz, Glycylcyclin-Resistenz, Cephalosporin-Resistenz, Ciprofloxacin-Resistenz, Nitrofurantoin-Resistenz, Trimethoprim/Sulfa-Resistenz, Piperacillin/Tazobactam-Resistenz, Moxifloxacin-Resistenz, Vancomycin-Resistenz, Teicoplanin-Resistenz, Penicillin-Resistenz und Makrolid-Resistenz.

33. Verwendung gemäß Anspruch 32, wobei die Glykopeptid-Resistenz eine Vancomycin-Resistenz ist.

34. Verwendung gemäß Anspruch 28, wobei der Krankheitserreger ausgewählt ist aus der Gruppe bestehend aus *Staphylococcus aureus, Staphylococcus epidermidis, Streptococcus pneumoniae* oder *Streptococcus pyogenes.*

35. Verwendung gemäß Anspruch 34, wobei die Infektion *Staphylococcus aureus* umfasst.

36. Verwendung gemäß Anspruch 35, wobei der *Staphylococcus aureus* eine Antibiotikum-Resistenz besitzt, die ausgewählt ist aus der Gruppe bestehend aus Glykopeptid-Resistenz, Tetracyclin-Resistenz, Minocyclin-Resistenz, Methicillin-Resistenz, Vancomycin-Resistenz und Resistenz gegenüber einem Glycylcyclin-Antibiotikum, das nicht Tigecyclin ist.

37. Verwendung gemäß Anspruch 28, wobei die Infektion *Acinetobacter baumannii* umfasst.

38. Verwendung gemäß Anspruch 37, wobei der *Acinetobacter baumannii* eine Antibiotikum-Resistenz besitzt, die ausgewählt ist aus der Gruppe bestehend aus Cephalosporin-Resistenz, Ciprofloxacin-Resistenz, Nitrofurantoin-Resistenz, Trimethoprim/Sulfa-Resistenz und Piperacillin/Tazobactam-Resistenz.

39. Verwendung gemäß Anspruch 28, wobei die Infektion *Mycobacterium abscessus* umfasst.

40. Verwendung gemäß Anspruch 39, wobei *Mycobacterium abscessus* Moxifloxacinresistent ist.

41. Verwendung gemäß Anspruch 28, wobei die Infektion einen Krankheitserreger umfasst, der aus der Gruppe bestehend aus *Haemophilus influenzae, Enterococcus faecium, Escherichia coli, Neisseria gonorrhoeae, Rickettsia prowazekii, Rickettsia typhi* oder *Rickettsia rickettsii* ausgewählt ist.

42. Verwendung gemäß Anspruch 27, wobei die Gelenkinfektion oder die Infektion der das Gelenk umgebenden Gewebe eine septische Arthritis verursacht.

43. Verwendung gemäß Anspruch 1 oder 2, wobei die Infektion des Knochens oder Knochenmarks eine Osteomyelitis verursacht.

44. Verwendung gemäß Anspruch 24 oder 25, wobei die Gelenkinfektion oder die Infektion der das Gelenk umgebenden Gewebe eine septische Arthritis verursacht.

## Revendications

1. Utilisation d'une quantité pharmacologiquement efficace de tigécycline pour la préparation d'un médicament pour le traitement d'une infection de l'os ou de la moelle osseuse chez un mammifère.

2. Utilisation selon la revendication 1, dans laquelle le traitement comprend de plus l'administration d'un agent antimicrobien choisi parmi le groupe constitué de la rifamycine, la rifampine, la rifapentine, la rifaximine, ou la streptovaricine.

3. Utilisation selon la revendication 2 où l'antimicrobien est la rifampine.

4. Utilisation selon la revendication 1, 2 ou 3 où l'infection comprend un pathogène choisi parmi le groupe constitué des bactéries Gram-négatives, des bactéries Gram-positives, des bactéries anaérobies, et des bactéries aérobies.

5. Utilisation selon la revendication 4 où le pathogène est choisi parmi le groupe constitué de *Staphylococcus, Acinetobacter, Mycobacterium, Haemophilus, Salmonella, Streptococcus, Enterobacteriaceae, Enterococcus, Escherichia, Pseudomonas, Neisseria, Rickettsia, Pneumococci, Prevotella, Peptostreptococci, Bacteroides Legionella, streptocoques bêta-hémolytiques,* streptocoque du groupe B et *spirochaetes.*

6. Utilisation selon la revendication 5 dans laquelle l'infection est constituée de *Neisseria, Mycobacterium, Staphylococcus,* et *Haemophilus.*

7. Utilisation selon la revendication 6 dans laquelle l'infection est constituée de *Neisseria meningitidis, Mycobacterium tuberculosis, Mycobacterium leprae, Staphylococcus aureus, Staphylococcus epidermidis,* ou *Haemophilus influenzae.*

8. Utilisation selon la revendication 4 où le pathogène présente une résistance antibiotique.

9. Utilisation selon la revendication 8 où la résistance antibiotique est choisie parmi le groupe constitué de la résistance à la méthicilline, la résistance aux glycopeptides, la résistance à la tétracycline, la résistance à l'oxytétracycline, la résistance à la doxycycline ; la résistance à la chlortétracycline, la résistance à la minocycline, la résistance à la glycylcycline, la résistance à la céphalosporine, la résistance à la ciprofloxacine, la résistance à la nitrofurantoïne, la résistance au triméthoprim-sulfa, la résistance à la pipéracilline/tazobactam, la résistance à la moxifloxacine, la résistance à la vancomycine, la résistance à la téicoplanine, la résistance à la pénicilline, et la résistance aux macrolides.

10. Utilisation selon la revendication 9 où la résistance aux glycopeptides est la résistance à la vancomycine.

11. Utilisation selon la revendication 5 où le pathogène est choisi parmi le groupe constitué de *Staphylococcus aureus, Staphylococcus epidermidis, Streptococcus pneumoniae,* ou *Streptococcus pyogenes.*

12. Utilisation selon la revendication 11 où l'infection est constituée de *Staphylococcus aureus.*

13. Utilisation selon la revendication 12 où *Staphylococcus aureus* présente une résistance antibiotique choisie parmi le groupe constitué de la résistance aux glycopeptides, la résistance à la tétracycline, la résistance à la minocycline, la résistance à la méthicilline, la résistance à la vancomycine, et la résistance à un antibiotique glycylcycline autre que la tigécycline.

14. Utilisation selon la revendication 5 où l'infection est constituée d'*Acinetobacter baumannii.*

15. Utilisation selon la revendication 14 où *Acinetobacter baumannii* présente une résistance antibiotique choisie parmi le groupe constitué de la résistance à la céphalosporine, la résistance à la ciprofloxacine, la résistance à la nitrofurantoïne, la résistance au triméthoprim-sulfa, et la résistance à la pipéracilline/tazobactam.

16. Utilisation selon la revendication 5 où l'infection est constituée de *Mycobacterium abscessus.*

17. Utilisation selon la revendication 16 où *Mycobacterium abscessus* présente une résistance à la moxifloxacine.

18. Utilisation selon la revendication 5 où l'infection est constituée de *Haemophilus influenzae.*

19. Utilisation selon la revendication 5 où l'infection est constituée de *Enterococcus faecium.*

20. Utilisation selon la revendication 5 où l'infection est constituée de *Escherichia coli.*

21. Utilisation selon la revendication 5 où l'infection est constituée de *Neisseria gonorrhoeae.*

22. Utilisation selon la revendication 5 où l'infection est constituée de *Rickettsia prowazekii, Rickettsia typhi,* ou *Rickettsia rickettsii.*

23. Utilisation selon la revendication 4 dans laquelle l'infection provoque une ostéomyélyte.

24. Utilisation d'une quantité pharmacologiquement efficace de tigécycline pour la préparation d'un médicament pour le traitement d'une infection de l'articulation ou d'une infection des tissus entourant l'articulation chez un mammifère.

25. Utilisation selon la revendication 24, dans laquelle le traitement comprend de plus l'administration d'un agent antimicrobien choisi parmi le groupe constitué de la rifamycine, la rifampine, la rifapentine, la rifaximine, ou la streptovaricine.

26. Utilisation selon la revendication 25 où l'antimicrobien est la rifampine.

27. Utilisation selon la revendication 24, 25 ou 26 où l'infection est constituée d'un pathogène choisi parmi le groupe constitué des bactéries Gram-négatives, des bactéries Gram-positives, des bactéries anaérobies, et des bactéries aérobies.

28. Utilisation selon la revendication 27 où le pathogène est choisi parmi le groupe constitué de *Staphylococcus, Acinetobacter, Mycobacterium, Haemophilus, Salmonella, Streptococcus, Enterobacteriaceae, Enterococcus, Escherichia, Pseudomonas, Neisseria, Rickettsia, Pneumococci, Prevotella, Peptostreptococci, Bacteroides Legionella, streptocoques bêta-hémolytiques,* streptocoque du groupe B et *spirochaetes.*

29. Utilisation selon la revendication 28 dans laquelle l'infection est constituée de *Neisseria, Mycobacterium, Staphylococcus,* et *Haemophilus.*

30. Utilisation selon la revendication 29 dans laquelle l'infection est constituée de *Neisseria meningitidis, Mycobacterium tuberculosis, Mycobacterium leprae, Staphylococcus aureus, Staphylococcus epidermidis,* ou *Haemophilus influenzae.*

31. Utilisation selon la revendication 27 où le pathogène présente une résistance antibiotique.

32. Utilisation selon la revendication 31 où la résistance antibiotique est choisie parmi le groupe constitué de la résistance à la méthicilline, la résistance aux glycopeptides, la résistance à la tétracycline, la résistance à l'oxytétracycline, la résistance à la doxycycline ; la résistance à la chlortétracycline, la résistance à la minocycline, la résistance à la glycylcycline, la résistance à la céphalosporine, la résistance à la ciprofloxacine, la résistance à la nitrofurantoïne, la résistance au triméthoprim-sulfa, la résistance à la pipéracilline/tazobactam, la résistance à la moxifloxacine, la résistance à la vancomycine, la résistance à la téicoplanine, la résistance à la pénicilline, et la résistance aux macrolides.

33. Utilisation selon la revendication 32 où la résistance aux glycopeptides est la résistance à la vancomycine.

34. Utilisation selon la revendication 28 où le pathogène est choisi parmi le groupe constitué de *Staphylococcus aureus, Staphylococcus epidermidis, Streptococcus pneumoniae,* ou *Streptococcus pyogenes.*

35. Utilisation selon la revendication 34 où l'infection est constituée de *Staphylococcus aureus.*

36. Utilisation selon la revendication 35 où *Staphylococcus aureus* présente une résistance antibiotique choisie parmi le groupe constitué de la résistance aux glycopeptides, la résistance à la tétracycline, la résistance à la minocycline, la résistance à la méthicilline, la résistance à la vancomycine, et la résistance à un antibiotique glycylcycline autre que la tigécycline.

37. Utilisation selon la revendication 28 où l'infection est constituée d'*Acinetobacter baumannii.*

38. Utilisation selon la revendication 37 où *Acinetobacter baumannii* présente une résistance antibiotique choisie parmi le groupe constitué de la résistance à la céphalosporine, la résistance à la ciprofloxacine, la résistance à la nitrofurantoïne, la résistance au triméthoprim-sulfa, et la résistance à la pipéracilline/tazobactam.

39. Utilisation selon la revendication 28 où l'infection est constituée de *Mycobacterium abscessus.*

40. Utilisation selon la revendication 39 où *Mycobacterium abscessus* présente une résistance à la moxifloxacine.

41. Utilisation selon la revendication 28 où l'infection est constituée d'un pathogène choisi parmi le groupe constitué de *Haemophilus influenzae, Enterococcus faecium, Escherichia coli, Neisseria gonorrhoeae,Rickettsia prowazekii, Rickettsia typhi,* ou *Rickettsia rickettsii.*

42. Utilisation selon la revendication 27 dans laquelle l'infection de l'articulation ou l'infection des tissus entourant l'articulation provoque une arthrite sceptique.

43. Utilisation selon la revendication 1 ou 2, dans laquelle l'infection de l'os ou de la moelle osseuse provoque une ostéomyélite.

44. Utilisation selon la revendication 24 ou 25 dans laquelle l'infection de l'articulation ou l'infection des tissus entourant l'articulation provoque une arthrite sceptique.
